(19) Europäisches Patentamt
European Patent Office
Office européen des brevets

(11) Veröffentlichungsnummer: **0 592 949 A2**

(12) # EUROPÄISCHE PATENTANMELDUNG

(21) Anmeldenummer: **93116244.0**

(22) Anmeldetag: **07.10.93**

(51) Int. Cl.[5]: **C07D 295/215**, C07D 213/75, A61K 31/56, A61K 31/445

(30) Priorität: **12.10.92 DE 4234295**

(43) Veröffentlichungstag der Anmeldung: **20.04.94 Patentblatt 94/16**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach(DE)**

(72) Erfinder: **Pieper, Helmut Dr.**
**kapellenweg 5**
**D-88440 Biberach(DE)**
Erfinder: **Linz, Günter Dr.**
**Erlenweg 8**
**D-88441 Mittelbiberach(DE)**
Erfinder: **Himmelsbach, Frank Dr.**
**Ahornweg 16**
**D-88441 Mittelbiberach(DE)**
Erfinder: **Austel, Volkhard Dr.**
**Kapellenweg 7**
**D-88400 Biberach(DE)**
Erfinder: **Müller, Thomas Dr.**
**Alter Postplatz 17**
**D-88400 Biberach(DE)**
Erfinder: **Weisenberger, Johannes Dr.**
**Haydnweg 5**
**D-88400 Biberach(DE)**
Erfinder: **Guth, Brian Dr.**
**Am Schlegelberg 24**
**D-88447 Warthausen(DE)**

(54) **N-(Aminocarbonyl-) Piperidin/Piperazin-Derivate als aggregationshemmende Wirkstoffe.**

(57) Die Erfindung betrifft Carbonsäurederivate der allgemeinen Formel

A - B - C - D - E - F - G ,(I)

in der
A bis G wie im Anspruch 1 definiert sind, deren Tautomere, deren Stereoisomere einschließlich deren Gemische und deren Additionssalze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung.

EP 0 592 949 A2

Die Erfindung betrifft Carbonsäurederivate der allgemeinen Formel

A - B - C - D - E - F - G ,(I)

deren Tautomere, deren Stereoisomere einschließlich deren Gemische und deren Additionssalze, insbesondere deren physiologisch verträgliche Additionssalze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel und deren Verwendung sowie Verfahren zu ihrer Herstellung.

In der obigen allgemeinen Formel I bedeutet

A eine geradkettige oder verzweigte Aminoalkylgruppe, eine Amino-, Amidino- oder Guanidinogruppe, wobei in jeder der vorstehend erwähnten Gruppen jeweils an einem der Stickstoffatome ein oder zwei Wasserstoffatome durch eine Alkyl- oder Phenylalkylgruppe oder ein Wasserstoffatom durch eine Alkoxycarbonylgruppe mit insgesamt 2 bis 5 Kohlenstoffatomen, durch eine $R_1$-CO-O-($R_2$CH)-O-CO-, Benzyloxycarbonyl- oder Allyloxycarbonylgruppe ersetzt sein kann, wobei

$R_1$ eine Alkylgruppe mit 1 bis 5 Kohlenstoffatomen, eine Cycloalkylgruppe mit 5 bis 7 Kohlenstoffatomen, eine Phenyl- oder Phenylalkylgruppe und

$R_2$ ein Wasserstoffatom, eine Alkylgruppe mit 1 bis 4 Kohlenstoffatomen, eine Cyloalkylgruppe mit 5 bis 7 Kohlenstoffatomen oder eine Phenylgruppe darstellen,

B einen 6-gliedrigen Aromaten, der ein oder zwei Stickstoffatome enthalten und zusätzlich im Kohlenstoffgerüst durch eine Trifluormethylgruppe monosubstituiert oder durch Fluor-, Chlor- oder Bromatome oder Alkylgruppen, wobei die Substituenten gleich oder verschieden sein können, mono- oder disubstituiert sein kann,

C eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sein können, oder eine 1,4-Cyclohexylengruppe, in der die Methylengruppe in 2-Stellung oder die Methylengruppen in 2- und 5-Stellung jeweils durch eine -$NR_3$-Gruppe ersetzt sind, wobei in einem so erhaltenen Ring, der ein oder zwei Stickstoffatome oder eine oder zwei -$NR_3$-Gruppen enthält, jeweils eine zu einem Stickstoffatom benachbarte Methylengruppe durch eine Carbonylgruppe ersetzt sein kann, oder eine 3,4-Dehydro-1,4-piperidinylengruppe, wobei

$R_3$ ein Wasserstoffatom, eine Alkyl- oder Phenylalkylgruppe darstellt,

D eine Methylen-, Ethylen-, Carbonyl- oder Methylencarbonylgruppe, wobei die Carbonylgruppe der Methylencarbonylgruppe mit dem Rest E verknüpft ist, oder eine -$NR_3$-CO-X-Gruppe, in der das Stickstoffatom der -$NR_3$-CO-X-Gruppe mit dem Rest C verknüpft ist, $R_3$ wie vorstehend erwähnt definiert ist und

X eine geradkettige oder verzweigte Alkylengruppe mit 1 bis 5 Kohlenstoffatomen oder eine 1,4-Cyclohexylengruppe darstellt,

E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch ein Stickstoffatom oder durch eine >C=CH-Gruppe ersetzt sein kann, oder eine -$NR_4$-X-Gruppe, in der

X wie vorstehend erwähnt definiert ist und

$R_4$ ein Wasserstoffatom, eine Alkylgruppe mit 1 bis 6 Kohlenstoffatomen, welche in 1-, 2- oder 3-Stellung durch eine Morpholinocarbonyl-, Pyrrolidinocarbonyl-, Piperidinocarbonyl- oder Hexamethyleniminocarbonylgruppe oder durch eine ($R_1NR_2$)-CO-Gruppe substituiert sein kann, wobei $R_1$ und $R_2$ wie vorstehend erwähnt definiert sind, oder eine Phenylalkylgruppe mit 1 bis 4 Kohlenstoffatomen im Alkylteil darstellen,

F eine Alkylengruppe oder auch, falls E keine 1,4-Piperazinylengruppe darstellt, eine Bindung und

G eine durch eine $R_5$O-Gruppe substituierte Carbonylgruppe, in der

$R_5$ ein Wasserstoffatom, eine Alkylgruppe mit 1 bis 6 Kohlenstoffatomen, die in 1-, 2-, 3-Stellung durch eine Morpholinocarbonyl-, Pyrrolidinocarbonyl-, Piperidinocarbonyl-, Hexamethyleniminocarbonyl- oder ($R_1NR_2$)-CO-Gruppe oder in 2- oder 3-Stellung durch eine Morpholino- oder Pyrrolidinon-1-yl-Gruppe substituiert sein kann, wobei $R_1$ und $R_2$ wie vorstehend erwähnt definiert sind, eine Phenylalkyl- oder Pyridylalkylgruppe, eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe mit 1 bis 4 Kohlenstoffatomen, durch eine Alkylgruppe mit 1 bis 4 Kohlenstoffatomen und durch 1 bis 3 Methylgruppen substituiert sein kann, eine Bicycloalkyl- oder Bicycloalkylalkylgruppe, in denen der Bicycloalkylteil jeweils 6 bis 10 Kohlenstoffatome enthält und zusätzlich durch 1 bis 3 Methylgruppen substituiert sein kann, darstellt,

eine Phosphono-, O-Alkylphosphono-, Tetrazol-5-yl- oder $R_6CO$-O-$CHR_2$-O-CO-Gruppe darstellen, wobei

$R_2$ wie vorstehend erwähnt definiert ist und

$R_6$ eine Alkylgruppe mit 1 bis 5 Kohlenstoffatomen, eine Cycloalkyl- oder Cycloalkoxygruppe mit jeweils 5 bis 7 Kohlenstoffatomen im Cycloalkylteil, eine Alkoxygruppe mit 1 bis 4 Kohlenstoffatomen, eine Phenyl-, Phenylalkyl- oder Phenylalkoxygruppe darstellt,

wobei, soweit nichts anderes erwähnt wurde,

die vorstehend erwähnten Alkyl-, Alkylen- oder Alkoxyteile jeweils 1 bis 3 Kohlenstoffatome enthalten können.

Bevorzugte Verbindungen der obigen allgemeinen Formel I sind jedoch diejenigen, in denen

A eine Aminoalkylgruppe mit 1 oder 2 Kohlenstoffatomen oder eine Amidinogruppe, wobei in den vorstehend erwähnten Gruppen an einem Stickstoffatom ein Wasserstoffatom durch eine Alkoxycarbonylgruppe mit insgesamt 2 oder 3 Kohlenstoffatomen, durch eine Benzyloxycarbonyl-, Allyloxycarbonyl- oder $R_1$-CO-O-($R_2$CH)-O-CO-Gruppe ersetzt sein kann, wobei

$R_1$ eine Alkylgruppe mit 1 oder 2 Kohlenstoffatomen und

$R_2$ ein Wasserstoffatom oder eine Alkylgruppe mit 1 oder 2 Kohlenstoffatomen darstellen,

B einen 6-gliedrigen Aromaten, der ein oder zwei Stickstoffatome enthalten und zusätzlich im Kohlenstoffgerüst durch ein Fluor-, Chlor- oder Bromatom oder durch eine Methylgruppe substituiert sein kann,

C eine 3,4-Dehydro-1,4-piperidinylengruppe, eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sein können, oder eine 2-Oxo-1,4-piperidinylengruppe,

D eine Methylen-, Ethylen-, Carbonyl- oder Methylencarbonylgruppe, wobei die Carbonylgruppe der Methylencarbonylgruppe mit dem Rest E verknüpft ist, oder eine -$NR_3$-CO-X-Gruppe, in der das Stickstoffatom der -$NR_3$-CO-X-Gruppe mit dem Rest C verknüpft ist,

$R_3$ ein Wasserstoffatom, eine Alkyl- oder Phenylalkylgruppe mit jeweils 1 bis 3 Kohlenstoffatomen im Alkylteil und

X eine geradkettige oder verzweigte Alkylengruppe mit 1 bis 4 Kohlenstoffatomen oder eine 1,4-Cyclohexylengruppe darstellen,

E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch ein Stickstoffatom oder durch eine >C=CH-Gruppe ersetzt sein kann, oder eine -$NR_4$-X-Gruppe, in der

X wie vorstehend erwähnt definiert ist und

$R_4$ ein Wasserstoffatom, eine Alkylgruppe mit 1 bis 6 Kohlenstoffatomen, eine Phenylalkylgruppe mit 1 bis 4 Kohlenstoffatomen im Alkylteil oder eine Morpholinocarbonylmethylgruppe darstellen,

F eine Methylengruppe oder auch, falls E keine 1,4-Piperazinylengruppe darstellt, eine Bindung und

G eine durch eine $R_5$O-Gruppe substituierte Carbonylgruppe, in der

$R_5$ ein Wasserstoffatom, eine Alkylgruppe mit 1 bis 6 Kohlenstoffatomen, die in 1-Stellung durch eine Morpholinocarbonyl-, Piperidinocarbonyl- oder Dimethylaminocarbonylgruppe und in 2-Stellung durch eine Morpholino- oder Pyrrolidinon-1-yl-Gruppe substituiert sein kann, eine Phenylmethyl- oder Pyridylmethylgruppe, eine Cycloalkyl- oder Cycloalkylmethylgruppe mit jeweils 5 oder 6 Kohlenstoffatomen im Cycloalkylteil, eine Menthyl- oder Norbornylgruppe darstellt,

oder eine $R_6CO$-O-$CHR_2$-O-CO-Gruppe darstellen, wobei

$R_2$ wie vorstehend erwähnt definiert ist und

$R_6$ eine Alkylgruppe mit 1 bis 4 Kohlenstoffatomen, eine Methoxy- oder Ethoxygruppe darstellt,

bedeuten,

deren Tautomere, deren Stereoisomere einschließlich deren Gemische und deren Additionssalze.

Besonders bevorzugte Verbindungen der obigen allgemeinen Formel I sind jedoch diejenigen, in denen

A eine Aminomethyl- oder eine Amidinogruppe, wobei in der Amidinogruppe an einem Stickstoffatom ein Wasserstoffatom durch eine Methoxycarbonyl-, Ethoxycarbonyl- oder Benzyloxycarbonylgruppe ersetzt sein kann,

B eine gegebenenfalls durch ein Bromatom substituierte Phenylengruppe oder eine Pyridinylengruppe,

C eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sein können,

D eine Methylen-, Ethylen-, Carbonyl- oder Methylencarbonylgruppe, wobei die Carbonylgruppe der Methylencarbonylgruppe mit dem Rest E verknüpft ist, oder eine -$NR_3$-CO-X-Gruppe, in der das Stickstoffatom der -$NR_3$-CO-X-Gruppe mit dem Rest C verknüpft ist,

$R_3$ ein Wasserstoffatom oder eine Alkylgruppe mit 1 bis 3 Kohlenstoffatomen und

X eine geradkettige oder verzweigte Alkylengruppe mit 2 oder 3 Kohlenstoffatomen oder eine 1,4-Cyclohexylengruppe darstellen,
E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch eine >C=CH-Gruppe ersetzt sein kann, oder eine -$NR_4$-X-Gruppe, in der

X wie vorstehend erwähnt definiert ist und

$R_4$ ein Wasserstoffatom, eine Alkylgruppe mit 1 bis 5 Kohlenstoffatomen oder eine Phenylalkylgruppe mit 1 bis 3 Kohlenstoffatomen im Alkylteil darstellen,
F eine Bindung oder eine Methylengruppe und
G eine durch eine $R_5$O-Gruppe substituierte Carbonylgruppe, in der

$R_5$ ein Wasserstoffatom, eine Alkylgruppe mit 1 bis 5 Kohlenstoffatomen, die in 1-Stellung durch eine Morpholinocarbonyl-, Piperidinocarbonyl- oder Dimethylaminocarbonylgruppe substituiert sein kann, oder eine Cycloalkylgruppe mit 5 oder 6 Kohlenstoffatomen darstellt,
bedeuten,
deren Tautomere, deren Stereoisomere einschließlich deren Gemische und deren Additionssalze.

Ganz besonders bevorzugte Verbindungen der obigen allgemeinen Formel I sind jedoch diejenigen, in denen
A und B zusammen eine 4-Aminomethyl-phenylgruppe, eine 4-Amidino-phenylgruppe, wobei in der Amidinogruppe an einem Stickstoffatom ein Wasserstoffatom durch eine Methoxycarbonyl- oder Benzyloxycarbonylgruppe ersetzt sein kann, oder eine 5-Amidino-pyrid-2-ylgruppe,
C eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sind,
D eine Methylen-, Ethylen-, Carbonyl- oder Methylencarbonylgruppe, wobei die Carbonylgruppe der Methylencarbonylgruppe mit dem Rest E verknüpft ist, oder eine -$NR_3$-CO-X-Gruppe, in der das Stickstoffatom der -$NR_3$-CO-X-Gruppe mit dem Rest C verknüpft ist,

$R_3$ ein Wasserstoffatom oder eine Methylgruppe und

X eine geradkettige oder verzweigte Alkylengruppe mit 2 oder 3 Kohlenstoffatomen oder eine 1,4-Cyclohexylengruppe darstellen,
E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch eine >C=CH-Gruppe ersetzt sein kann, oder eine -$NR_4$-X-Gruppe, in der

X wie vorstehend erwähnt definiert ist und

$R_4$ ein Wasserstoffatom, eine geradkettige Alkylgruppe mit 1 bis 5 Kohlenstoffatomen, eine Benzyl-, 2-Phenylethyl- oder 3-Phenyl-propylgruppe darstellen,
F eine Bindung oder eine Methylengruppe und
G eine durch eine $R_5$O-Gruppe substituierte Carbonylgruppe, in der

$R_5$ ein Wasserstoffatom, eine Alkylgruppe mit 1 bis 4 Kohlenstoffatomen, eine Methylgruppe, die durch eine Morpholinocarbonyl- oder Dimethylaminocarbonylgruppe substituiert ist, oder eine Cyclohexylgruppe darstellt,
bedeuten,
deren Stereoisomere einschließlich deren Gemische und deren Additionssalze,
insbesondere jedoch die Verbindungen

(a) 1-(4-Amidinophenyl)-4-[N-(trans-4-carboxycyclohexyl)-aminocarbonyl]-piperazin,
(b) 1-(4-Amidinophenyl)-4-[N-(trans-4-carboxycyclohexyl)-N-methyl-aminocarbonyl]-piperazin,
(c) 1-(5-Amidinopyrid-2-yl)-4-[N-(trans-4-carboxycyclohexyl)-aminocarbonyl]-piperazin,
(d) 4-(4-Amidinophenyl)-1-[N-(trans-4-carboxycyclohexyl)-aminocarbonyl]-piperidin,
(e) 1-(4-Amidinophenyl)-4-[N-benzyl-N-(trans-4-carboxycyclohexyl)-aminocarbonyl]-piperazin,
(f) 1-(5-Amidinopyrid-2-yl)-4-[N-(trans-4-carboxycyclohexyl]-aminocarbonyl]-piperazin,
(g) 1-(5-Amidinopyrid-2-yl)-4-[N-(trans-4-carboxycyclohexyl]-N-methyl-aminocarbonyl]-piperazin,
(h) 4-(4-Aminomethylphenyl)-1-[N-(trans-4-carboxycyclohexyl)-aminocarbonyl]-piperidin,
(i) 4-(4-Amidinophenyl)-4-[4-(carboxymethyl)-piperidinocarbonyl]-piperazin,
(j) 1-(4-Amidinophenyl)-4-[4-(carboxymethyl)-3,4-dehydropiperidinocarbonyl]-piperazin,
(k) 1-(5-Aminomethylpyrid-2-yl)-4-[N-(trans-4-carboxycyclohexyl)-aminocarbonyl]-piperazin,
(l) 1-(5-Amidinopyrid-2-yl)-4-[4-(carboxymethyl)-piperidinocarbonyl]-piperidin,
(m) 1-(4-Aminomethylphenyl)-4-[N-(trans-4-carboxycyclohexyl)-aminocarbonyl]-piperazin,
(n) 1-[4-(N-Benzyloxycarbonyl-amidino)-phenyl]-4-[N-(trans-4-carboxycyclohexyl)-aminocarbonyl]-piperazin,

(o) 4-(4-Amidinophenyl)-1-[N-(trans-4-carboxycyclohexyl)-aminocarbonyl]-piperidin,
(p) 1-(4-Amidinophenyl)-4-[4-(carboxymethyl)-piperidinocarbonyl]-piperidin,
(q) 1-(4-Amidinophenyl)-4-[N-(2-carboxyethyl)-aminocarbonylmethyl]-piperidin,
(r) 1-(4-Amidinophenyl)-4-[N-(trans-4-carboxycyclohexyl)-aminocarbonyl]-piperidin,
(s) 1-(4-Aminomethylphenyl)-4-[N-(trans-4-carboxycyclohexyl)-aminocarbonyl]-piperidin,
(t) 1-(4-Amidinophenyl)-4-[N-(trans-4-carboxycyclohexyl)-N-(2-phenylethyl)-aminocarbonyl]-piperidin,
(u) 1-(4-Amidinophenyl)-4-[N-(trans-4-carboxycyclohexyl)-N-(3-phenylpropyl)-aminocarbonyl]-piperidin und
(v) 1-(4-Amidinophenyl)-4-[N-(trans-4-carboxycyclohexyl)-N-(n-pentyl)-aminocarbonyl]-piperidin,

deren Ester mit Cyclohexanol oder mit einem Alkanol mit 1 bis 4 Kohlenstoffatomen, wobei das Methanol durch eine Morpholinocarbonyl- oder Dimethylaminocarbonylgruppe substituiert sein kann, deren Stereoisomere einschließlich deren Gemische und deren Additionssalze.

Erfindungsgemäß erhält man die neuen Verbindungen der allgemeinen Formel I beispielsweise nach folgenden Verfahren:

a) Zur Herstellung von Verbindungen der allgemeinen Formel I, in der G eine Carboxygruppe darstellt: Überführung einer Verbindung der allgemeinen Formel

A - B - C - D - E - F - G' ,(II)

in der
A, B, C, D, E und F wie eingangs definiert sind und
G', das an ein Kohlenstoffatom gebunden ist, eine mittels Hydrolyse, Behandlung mit Säuren, Thermolyse oder Hydrogenolyse in eine Carboxygruppe überführbare Gruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert. Butylester, mittels Behandlung mit einer Säure oder Thermolyse in eine Carboxylgruppe, Ester mit Aralkanolen, z.B. der Benzylester, mittels Hydrogenolyse in eine Carboxylgruppe, und Bis(alkoxycarbonyl)-methylgruppen mittels Hydrolyse oder Behandlung mit einer Säure in eine Bis(hydroxycarbonyl)-methylgruppe, welche anschließend decarboxyliert wird, übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Methanol, Wasser/Methanol, Äthanol, Wasser/Äthanol, Wasser/Isopropanol, Wasser/Tetrahydrofuran oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet G' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50 °C in die Carboxylgruppe übergeführt werden.

Bedeutet G' in einer Verbindung der Formel II beispielsweise die tert. Butyloxycarbonylgruppe, so kann die tert. Butylgruppe auch durch Behandlung mit einer Säure wie Trifluoressigsäure, Salzsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 °C und 120 °C, z.B. bei Temperaturen zwischen 0 und 60 °C, oder auch thermisch gegebenenfalls in einem inerten Lösungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet G' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 10 bar abgespalten werden. Bei der Hydrogenolyse können

gleichzeitig andere Reste, z.B. eine Nitrogruppe zur Aminogruppe oder eine Benzyloxygruppe zur Hydroxygruppe mitreduziert oder eine Benzyloxycarbonylamidinogruppe in die Amidinogruppe übergeführt werden.

b) Zur Herstellung von Verbindungen der allgemeinen Formel I, in der A eine Amidinogruppe darstellt, in der an einem der Stickstoffatome ein oder zwei Wasserstoffatome durch eine Alkyl- oder Phenylalkylgruppe ersetzt sein können:

Umsetzung einer gegebenenfalls im Reaktionsgemisch gebildeten Verbindung der allgemeinen Formel

$Z_1$ - C(=NH) - B - C - D - E - F - G ,(III)

in der

B, C, D, E, F und G wie eingangs definiert sind und

$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$R_a$ - NH - $R_b$ ,(IV)

in der

$R_a$ und $R_b$, die gleich oder verschieden sein können, Wasserstoffatome, Alkyl- oder Phenylalkylgruppen mit jeweils 1 bis 3 Kohlenstoffatome im Alkylteil bedeuten.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150 °C, vorzugsweise bei Temperaturen zwischen 20 und 120 °C, mit einem entsprechenden freien Amin oder mit einem entsprechenden Säureadditionssalz wie beispielsweise Ammoniumcarbonat oder Ammoniumacetat durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder in Gegenwart eines entsprechenden Alkoholats wie Natriummethylat oder Natriumethylat oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50 °C, vorzugsweise jedoch bei Temperaturen zwischen 0 und 20 °C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

c) Zur Herstellung von Verbindungen der allgemeinen Formel I, in der A eine Amidinogruppe darstellt:

Umsetzung einer Verbindung der allgemeinen Formel

NC - B - C - D - E - F - G ,(V)

in der

B, C, D, E, F und G wie eingangs definiert sind, mit Hydroxylamin und anschließender Reduktion des so erhaltenen Amidoxims.

Die Umsetzung mit Hydroxylamin wird zweckmäßigerweise in einem Lösungsmittel wie Methanol, Ethanol, n-Propanol, Wasser, Methanol/Wasser, Ethanol/Wasser, Tetrahydrofuran oder Dioxan gegebenenfalls unter Zusatz einer Base wie z. B. Natriumcarbonat bei Temperaturen zwischen 0 und 100 °C, vorzugsweise jedoch bei Temperaturen zwischen 20 und 80 °C entweder mit freiem Hydroxylamin oder mit einem entsprechenden Säureadditionssalz wie beispielsweise dem Hydrochlorid durchgeführt.

Die nachfolgende Reduktion wird vorzugsweise in einem geeigneten Lösungsmittel wie Methanol, Methanol/Wasser, Methanol/Ammoniak, Methanol/Wasser/Ammoniak, Methanol/Salzsäure, Ethanol, Ether, Tetrahydrofuran, Dioxan, Dimethylformamid oder Eisessig in Gegenwart von katalytisch angeregtem Wasserstoff, z.B. von Wasserstoff in Gegenwart von Raney-Nickel, Platin oder Palladium/Kohle bei Temperaturen zwischen 0 und 100 °C, vorzugsweise bei Temperaturen zwischen 20 und 80 °C, durchgeführt.

d) Zur Herstellung von Verbindungen der allgemeinen Formel I, in der A eine Amidinogruppe darstellt, in der an einem der Stickstoffatome ein oder zwei Wasserstoffatome durch eine Alkyl- oder Phenylalkylgruppe ersetzt sein können:

Umsetzung einer Verbindung der allgemeinen Formel

NC - B - C - D - E - F - G ,(V)

in der

B, C, D, E, F und G wie eingangs definiert sind, mit einem entsprechenden Alkylchloroaluminiumamid.

Die Umsetzung wird vorzugsweise in einem geeigneten Lösungsmittel, beispielsweise in Benzol oder Toluol, bei Temperaturen zwischen 0 und 100 °C, vorzugsweise jedoch bei einer Temperatur zwischen 20 und 80 °C durchgeführt und der so erhaltene Aluminiumkomplex anschließend hydrolytisch zersetzt, vorzugsweise mit Hilfe einer Aufschlämmung von Kieselgel in Chloroform (siehe R. S. Garigipati, Tetrahydron Letters 31, 1969 (1990)).

e) Zur Herstellung von Verbindungen der allgemeinen Formel I, in der A eine geradkettige oder verzweigte Aminoalkylgruppe mit 1 bis 3 Kohlenstoffatomen darstellt:

Reduktion einer Verbindung der allgemeinen Formel

$A_1$ - B - C - D - E - F - G ,(VI)

in der

B, C, D, E, F und G wie eingangs definiert sind und

$A_1$ eine Cyano-, Cyanomethyl-, 1-Cyanoethyl- oder 2-Cyanoethylgruppe darstellt.

Die Reduktion wird vorzugsweise in einem geeigneten Lösungsmittel wie Methanol, Methanol/Wasser, Methanol/Ammoniak, Methanol/Wasser/Ammoniak, Methanol/Salzsäure, Methanol/etherische Salzsäure, Ethanol, Ether, Tetrahydrofuran, Dioxan, Dimethylformamid oder Eisessig in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid oder Lithiumborhydrid bei Temperaturen zwischen 0 und 100 °C, vorzugsweise bei Temperaturen zwischen 20 und 80 °C, durchgeführt.

f) Zur Herstellung von Verbindungen der allgemeinen Formel I, in der A eine geradkettige oder verzweigte Aminoalkylgruppe mit 1 bis 3 Kohlenstoffatomen, eine Amino-, Amidino- oder Guanidinogruppe, wobei in jeder der vorstehend erwähnten Gruppen jeweils an einem der Stickstoffatome ein Wasserstoffatom durch eine Alkoxycarbonylgruppe mit insgesamt 2 bis 5 Kohlenstoffatomen, durch eine Benzyloxycarbonyl-, Allyloxycarbonyl- oder $R_1$-CO-O-($R_2$CH)-O-CO-Gruppe ersetzt ist, darstellt, wobei $R_1$ und $R_2$ wie eingangs definiert sind:

Umsetzung einer Verbindung der allgemeinen Formel

$A_2$ - B - C - D - E - F - G ,(VII)

in der

B, C, D, E, F und G wie eingangs definiert sind und

$A_2$ eine geradkettige oder verzweigte Aminoalkylgruppe mit 1 bis 3 Kohlenstoffatomen, eine Amino-, Amidino- oder Guanidinogruppe darstellt, mit einer Verbindung der allgemeinen Formel

$Z_2$ - $R_c$ ,(VIII)

in der

$R_c$ eine Alkoxycarbonylgruppe mit insgesamt 2 bis 5 Kohlenstoffatomen, eine Benzyloxycarbonyl-, Allyloxycarbonyl- oder $R_1$-CO-O-($R_2$CH)-O-CO-Gruppe, wobei $R_1$ und $R_2$ wie eingangs definiert sind, und

$Z_2$ eine nukleophile Austrittsgruppe wie ein Halogenatom, z. B. ein Chlor- oder Bromatom, oder eine gegebenenfalls substituierte Phenoxygruppe, z. B. eine p-Nitro-phenoxygruppe, darstellen.

Die Umsetzung wird zweckmäßigerweise in einem Lösungsmittel oder Lösungsmittelgemisch wie Wasser, Tetrahydrofuran, Tetrahydrofuran/Wasser, Dioxan, Dioxan/Wasser, Methylenchlorid, Chloroform, Essigester oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100 °C, vorzugsweise jedoch bei Temperaturen zwischen -10 und 80 °C, durchgeführt.

g) Zur Herstellung von Verbindungen der allgemeinen Formel I, in der G eine durch eine $R_5$'O-Gruppe substituierte Carbonylgruppe darstellt, wobei $R_5$' mit Ausnahme des Wasserstoffatoms die für $R_5$ eingangs erwähnten Bedeutungen besitzt:

Umsetzung einer Verbindung der allgemeinen Formel

A - B - C - D - E - F - $G_1$ ,(IX)

in der
A, B, C, D, E und F wie eingangs definiert sind und
$G_1$ eine Carboxy- oder Alkoxycarbonylgruppe darstellt, mit einem Alkohol der allgemeinen Formel

HO - $R_5'$ ,(X)

in der
$R_5'$ mit Ausnahme des Wasserstoffatoms die für $R_5$ eingangs erwähnten Bedeutungen besitzt.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid,Dimethylaminopyridin oder 1-Hydroxybenzotriazol, N,N'-Carbonyldiimidazol- oder N,N'-Thionyldiimidazol oder Triphenylphosphin/Tetrachlorkohlenstoff, zweckmäßigerweise bei Temperaturen zwischen 0 und 150°C, vorzugsweise bei Temperaturen zwischen 0 und 50°C, durchgeführt.

Die Umsetzung einer entsprechenden Alkoxyverbindung der allgemeinen Formel IX mit einem Alkohol der allgemeinen Formel X wird vorzugsweise in dem betreffenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 100°C, vorzugsweise bei Temperaturen zwischen 20 und 80°C, durchgeführt.

h) Zur Herstellung von Verbindungen der allgemeinen Formel I, in der G eine durch eine $R_5'$O-Gruppe substituierte Carbonylgruppe oder eine $R_6$CO-O-CH$R_2$-O-CO-Gruppe darstellt, wobei $R_2$ und $R_6$ wie eingangs erwähnt definiert sind und $R_5'$ mit Ausnahme des Wasserstoffatoms die für $R_5$ eingangs erwähnten Bedeutungen besitzt:
Umsetzung einer Verbindung der allgemeinen Formel

A - B - C - D - E - F - COOH ,(XI)

in der
A, B, C, D, E und F wie eingangs definiert sind, mit einer Verbindung der allgemeinen Formel

$Z_3$ - $R_d$ ,(XII)

in der
$R_d$ mit Ausnahme des Wasserstoffatoms die für $R_5$ eingangs erwähnten Bedeutungen besitzt oder eine $R_6$CO-O-CH$R_2$-Gruppe darstellt, wobei $R_2$ und $R_6$ wie eingangs erwähnt definiert sind und
$Z_3$ eine nukleophile Austrittsgruppe wie ein Halogenatom, z. B. ein Chlor- oder Bromatom, darstellt.

Die Umsetzung wird zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat, Kaliumcarbonat oder Natronlauge oder in Gegenwart einer tertiären organischen Base wie N-Ethyldiisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 ° C, vorzugsweise bei Temperaturen zwischen 10 und 50 ° C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50 ° C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer-(IV)ammoniumnitrat in einem Lösungsmittel wie Methylenchlorid, Acetonitril oder Acetonitril/Wasser bei Temperaturen zwischen 0 und 50 ° C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 ° C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100 ° C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70 ° C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und chirale Verbindungen in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren 'siehe Beispiele I bis XXVI), z. B. durch nukleophile Substitutionsreaktionen an Aromaten (siehe Jerry March in Advanced Organic Chemistry, Third Edition, John Wiley & Sons, S. 576-578 (1985) und Bunnett and Zahler in Chem. Rev. 49, 273-412 (1951)).

Wie bereits eingangs erwähnt, weisen die neuen Carbonsäurederivate der allgemeinen Formel I und deren Additionssalze, insbesondere deren physiologisch verträgliche Additionssalze mit anorganischen oder organischen Säuren oder Basen, wertvolle Eigenschaften auf. So weisen die neuen Verbindungen der allgemeinen Formel I, in denen A eine gegebenenfalls substituierte Aminoalkyl-, Amino-, Amidino- oder Guanidinogruppe oder eine gegebenenfalls in vivo in eine Aminoalkyl-, Amino-, Amidino- oder Guanidinogruppe überführbare Gruppe, z.B. eine durch eine Alkoxycarbonyl, Benzyloxycarbonyl-, Allyloxycarbonyl- oder $R_1$-CO-O-($R_2$CH)-O-CO-Gruppe substituierte Aminoalkyl-, Amino-, Amidino- oder Guanidinogruppe, enthält und G eine Carboxyl-, Phosphono-, O-Alkyl-phosphono- oder 5-Tetrazolylgruppe oder eine gegebenenfalls in vivo in eine Carboxyl-, Phosphono-, O-Alkyl-phosphono- oder 5-Tetrazolylgruppe überführbare Gruppe, z.B. eine durch eine $R_5$O- oder $R_6$-CO-OCH$R_2$-O-Gruppe substituierte Carbonylgruppe, darstellt, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

1. Kompetitive Bindung $^3$H-BIBU 52/Testsubstanz an Humanthrombozyten:

Eine Suspension von Humanthrombozyten in Plasma wird mit $^3$H-BIBU 52 [= (3S,5S)-5-[(4'-Amidino-4-biphenylyl)oxymethyl]-3-[(carboxyl)methyl]-2-pyrrolidinon[3-$^3$H-4-biphenylyl]], das den literaturbekannten Liganden $^{125}$I-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 $\mu$l werden mit 50 $\mu$l physiologischer Kochsalzlösung, 100 $\mu$l Testsubstanzlösung, 50 $\mu$l $^{14}$C-Sucrose (3.700 Bq) und 50 $\mu$l $^3$H-BIBU 52 (Endkonzentration: 5 nM) bei Raumtemperatur 20 Minuten inkubiert. Zur Messung der unspezifischen Bindung wird anstelle der Testsubstanz 5 $\mu$l BIBU 52 (Endkonzentration: 30 $\mu$M) eingesetzt. Die Proben werden 20 Sekunden bei 10000 x g zentrifugiert und der Überstand abgezogen. 100 $\mu$l hiervon werden zur Bestimmung des freien Liganden gemessen. Das Pellet wird in 500 $\mu$l 0,2N NaOH gelöst, 450 $\mu$l werden mit 2 ml Szintillator und 25 $\mu$l 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. Antithrombotische Wirkung

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Konzentration wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37°C inkubiert. Aus den Konzentrations-Wirkungskurven wird die $EC_{50}$ berechnet, die die Konzentration beschreibt, bei der die Änderung der "optical density" halbmaximal gehemmt ist.

Die nachfolgende Tabelle enthält die gefundenen Ergebnisse:

| Substanz (Beispiel Nr.) | Kompetitive Bindung $^{3}$H-BIBU 52/Testsubstanz an Humanthrombozyten $IC_{50}$ [nM] | Hemmung der Plättchenaggregation $EC_{50}$[nM] |
|---|---|---|
| 1(1) | 870.0 | 110 |
| 1(5) | 1 200.0 | 80 |
| 1(9) | 17 000.0 | 90 |
| 1(24) | 4 400.0 | 220 |
| 1(25) | 30 000.0 | 6 800 |
| 2 | 7.4 | 40 |
| 2(1) | 18.0 | 40 |
| 2(2) | 47.0 | 90 |
| 2(3) | 4.5 | 30 |
| 2(6) | 1.5 | 40 |
| 2(7) | 29.0 | 80 |
| 2(8) | 27.0 | 60 |
| 2(11) | 230.0 | 380 |
| 2(17) | 44.0 | 130 |
| 2(19) | 370.0 | 620 |
| 2(22) | 310.0 | 680 |
| 2(42) | 100.0 | 170 |
| 3 | 130.0 | 230 |
| 3(1) | 230.0 | 660 |
| 3(2) | 14.0 | 100 |
| 3(4) | 78.0 | 100 |
| 3(12) | 180.0 | 290 |
| 3(15) | 5.7 | 30 |
| 3(17) | 74.0 | 180 |
| 3(24) | 9.1 | 110 |
| 3(25) | 4.0 | 52 |
| 3(26) | 12.0 | 65 |
| 4 | 5 300.0 | 4 300 |
| 7 | 1 200.0 | 280 |
| 7(2) | 2 100.0 | 360 |
| 8 | 540.0 | 190 |

Die Hemmung der Thrombozytenaggregation nach oraler Gabe der Prüfsubstanz wird ex vivo an Rhesusaffen ermittelt.

Direkt vor der oralen Verabreichung der in Natrosol suspendierten Prüfsubstanz wird den Tieren eine Blutprobe aus der Kubitalvene als Referenzwert entnommen. Zu definierten Zei- ten nach der Substanzgabe werden erneut Blutproben gewonnen und wie folgt untersucht.

Das mit 3,14% Natriumcitrat im Volumenverhältnis 1:10 versetzte Vollblut wird mit 200 g für 15 Minuten zentrifugiert. Der Überstand an plättchenreichem Plasma wird vorsichtig abgehoben. Aus dem erythrozytenreichen Sediment wird durch Zentrifugation mit 4000 g für 10 Minuten das plättchenarme Plasma als Überstand gewonnen.

Die mit Collagen (Hormonchemie, München; 2 µg/ml Endkonzentration im plättchenreichen Plasma) ausgelöste Thrombozytenaggregation in diesen ex vivo Proben wird nach der Methode von Born und Cross (J. Physiol. 170, 397 (1964)) photometrisch gemessen. Die nach Collagenstimulation gemessene größte Lichtdurchlässigkeit des plättchenreichen Plasmas wird mit dem Referenzwert verglichen, um die Hemmung der Aggregation zu den verschiedenen Zeitpunkten der Blutentnahme nach Substanzgabe relativ zum Referenzwert zu bestimmen.

Die Verbindung des Beispiels 1(24) hemmt die Collagen-induzierte Thrombozytenaggregation ex vivo nach oraler Gabe von 1 mg/kg länger als 4 Stunden.

Die erfindungsgemäßen Verbindungen sind gut verträglich, da beispielsweise bei intravenöser Gabe von 30 mg/kg an jeweils 3 Mäusen bei den Verbindungen der Beispiele 2, 2(7), 2(8), 3 und 5 keine Tieren gestorben waren.

Auf Grund ihrer Hemmwirkung auf Zell-Zell- bzw. Zell-Matrix-Wechselwirkungen eignen sich die neuen Carbonsäurederivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose, der Metastasierung von Tumoren und der Therapie genetisch bedingter oder auch erworbener Störungen der Interaktion von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Herstellung der Ausgangsprodukte:

Beispiel I

1-(4-Cyanophenyl)-piperazin

5,0 g 4-Fluorbenzoesäurenitril und 17,8 g Piperazin werden 4 Stunden bei 110°C gerührt. Das abgekühlte Gemisch wird in Wasser gelöst und die wässrige Phase mit Essigsäureester extrahiert. Die organische Phase wird mit gesättigter Kochsalzlösung gewaschen, über Natriumsulfat getrocknet und das Lösungsmittel unter vermindertem Druck entfernt. Der verbleibende Rückstand wird über Kieselgel chromatographiert.
Ausbeute: 7,0 g (91 % der Theorie),
$R_f$-Wert: 0,31 (Kieselgel; Methylenchlorid/Methanol/konz. Ammoniak = 9:1:0,1)

Beispiel II

1-(4-Cyanophenyl)-4-[[trans-4-(methoxycarbonyl)-cyclohexyl]-aminocarbonyl]-piperazin

Zu einer Suspension von 4,85 g Chlorameisensäure-p-nitrophenylester und 4,65 g trans-4-Aminocyclohexancarbonsäuremethylester-hydrochlorid in 200 ml Tetrahydrofuran tropft man bei 0°C eine Lösung von 10 ml Triethylamin in 50 ml Tetrahydrofuran. Man rührt 2,5 Stunden bei 0°C und gibt anschließend 4,5

g 1-(4-Cyanophenyl)-piperazin zu. Man rührt 16 Stunden bei Raumtemperatur und erwärmt 4 Stunden auf 50 °C. Unter vermindertem Druck werden ca. 200 ml Tetrahydrofuran abgedampft und der Rückstand in Essigester gelöst. Die organische Phase wird zweimal mit 1N Natronlauge und einmal mit gesättigter Kochsalzlösung gewaschen und über Natriumsulfat getrocknet.

Das Lösungsmittel wird unter vermindertem Druck entfernt und das Rohprodukt mit Essigester als Eluens über Kieselgel chromatographiert.
Ausbeute: 6,0 g (65 % der Theorie),
Schmelzpunkt: 177-179 °C
$R_f$-Wert: 0,67 (Kieselgel; Methylenchlorid/Methanol/konz. Ammoniak = 9:1:0,1)
Analog Beispiel II werden folgende Verbindungen erhalten:

(1) 1-(5-Cyanopyrid-2-yl)-4-[[trans-4-(methoxycarbonyl)-cyclohexyl]-aminocarbonyl]-piperazin
Es wird 1-(5-Cyanopyrid-2-yl)-piperazin eingesetzt.
Das Rohprodukt wird mit Essigester verrieben und der Niederschlag abgenutscht und getrocknet.
Schmelzpunkt: 176-179 °C
$R_f$-Wert: 0,50 (Kieselgel; Methylenchlorid/Methanol/konz. Ammoniak = 9:1:0,1)

(2) 4-(4-Cyanophenyl-1-[[trans-4-(methoxycarbonyl)-cyclohexyl]-aminocarbonyl]-piperidin
Es wird 4-(4-Cyanophenyl)-piperidin-hydrochlorid eingesetzt.
Schmelzpunkt: 136-138 °C
$R_f$-Wert: 0,32 (Kieselgel; Essigester/Cyclohexan = 4:1)

## Beispiel III

### 1-(4-Cyanophenyl)-4-[N-[trans-4-(methoxycarbonyl)-cyclohexyl]-N-methyl-aminocarbonyl]-piperazin

Eine Lösung von 2,50 g 1-(4-Cyanophenyl)-4-[[trans-4-(methoxycarbonyl)-cyclohexyl]-aminocarbonyl]-piperazin und 0,76 g Kalium-tert.butylat in 30 ml Dimethylsulfoxid wird 30 Minuten bei Raumtemperatur gerührt. Anschließend wird 0,5 ml Methyliodid zugetropft und 1,5 Stunden bei Raumtemperatur gerührt. Die Reaktionslösung wird mit 100 ml Wasser verdünnt und die wässrige Phase dreimal mit Essigester extrahiert. Die vereinigten organischen Phasen werden mit gesättigter Kochsalzlösung gewaschen und über Natriumsulfat getrocknet. Das Lösungsmittel wird unter vermindertem Druck entfernt und der verbleibende Rückstand mit Essigester/Cyclohexan (3:1) als Eluens über Kieselgel chromatographiert.
Ausbeute: 1,0 g (39 % der Theorie).
Das Produkt enthält ca. 20 % cis-Verbindung
$R_f$-Wert: 0,58 (Kieselgel; Essigester)
Als weitere Fraktion werden 1,0 g Ausgangsmaterial erhalten.
$R_f$-Wert: 0,45 (Kieselgel; Essigester)
Analog Beispiel III werden folgende Verbindungen erhalten:

(1) 1-(5-Cyanopyrid-2-yl)-4-[N-[trans-4-(methoxycarbonyl)-cyclohexyl]-N-methyl-aminocarbonyl]-piperazin
$R_f$-Wert: 0,56 (Kieselgel; Essigester)

(2) 4-(4-Cyanophenyl)-1-[N-[trans-4-(methoxycarbonyl)-cyclohexyl]-N-methyl-aminocarbonyl]-piperidin
$R_f$-Wert: 0,57 (Kieselgel; Essigester/Cyclohexan = 4:1)

(3) 1-(4-Cyanophenyl)-4-[N-(3-methoxycarbonylpropyl)-N-methyl-aminocarbonyl]-piperidin

(4) 1-(4-Cyanophenyl)-4-[N-(3-methoxycarbonylpropyl)-N-benzyl-aminocarbonyl]-piperidin
Unter Verwendung von Benzylbromid als Alkylierungsreagenz.

(5) 1-(4-Cyanophenyl)-4-[N-(3-methoxycarbonylpropyl)-N-(2-phenylethyl)-aminocarbonyl]-piperidin
Unter Verwendung von 2-Phenylethyliodid als Alkylierungsreagenz.

(6) 1-(4-Cyanophenyl)-4-[N-(3-methoxycarbonylpropyl)-N-(3-phenylpropyl)-aminocarbonyl]-piperidin
Unter Verwendung von 3-Phenylpropyliodid als Alkylierungsreagenz.

(7) 1-(4-Cyanophenyl)-4-[N-(3-methoxycarbonylpropyl)-N-(n-pentyl)-aminocarbonyl]-piperidin
Unter Verwendung von n-Pentyliodid als Alkylierungsreagenz.

(8) 1-(4-Cyanophenyl)-4-[N-(2-methoxycarbonylethyl)-N-methylaminocarbonylmethyl]-piperidin

(9) 1-(4-Cyanophenyl)-4-[N-(2-methoxycarbonylethyl)-N-(2-phenylethyl)-aminocarbonylmethyl]-piperidin
Unter Verwendung von 2-Phenylethyliodid

(10) 1-(4-Cyanophenyl)-4-[N-[trans-4-(methoxycarbonyl)-cyclohexyl]-N-methyl-aminocarbonyl]-piperidin

(11) 1-(4-Cyanophenyl)-4-[N-(2-methoxycarbonylethyl)-N-(2-phenylethyl)-aminocarbonylmethyl]-piperazin

(12) 1-(4-Cyanophenyl)-4-[(4-trans-methoxycarbonyl-cyclohexyl)-carbonylmethylamino]-piperidin

Beispiel IV

6-Bromnicotinsäurenitril

38,0 g 6-Chlornicotinsäureamid und 5,0 g Phosphoroxybromid werden 4 Stunden bei 100°C gerührt. Die Reaktionslösung wird portionsweise in 1 1 Wasser eingerührt. Der Niederschlag wird abgenutscht, mit Wasser gewaschen und getrocknet.
Ausbeute: 40,0 g (90 % der Theorie),
$R_f$-Wert: 0,91 (Kieselgel; Methylenchlorid/Methanol = 15:1)

Beispiel V

1-(5-Cyanopyrid-2-yl)-piperazin

0,6 g 6-Bromnicotinsäurenitril und 22,6 g Piperazin in 50 ml Dimethylformamid werden 2 Stunden bei 80°C gerührt. Das Lösungsmittel wird unter vermindertem Druck entfernt, der Rückstand in Wasser suspendiert und die wässrige Phase mit Essigester extrahiert. Die organische Phase wird mit gesättigter Kochsalzlösung gewaschen, über Natriumsulfat getrocknet und das Lösungsmittel unter vermindertem Druck entfernt. Der verbleibende Rückstand wird über Kieselgel chromatographiert. Ausbeute: 8,0 g (81 % der Theorie),
Schmelzpunkt: 73-75°C
$R_f$-Wert: 0,27 (Kieselgel; Methylenchlorid/Methanol/konz. Ammoniak = 9:1:0,1)

Beispiel VI

4-Carboxy-1-(5-cyanopyrid-2-yl)-piperidin

Eine Suspension aus 1,8 g 6-Bromnicotinsäurenitril, 1,3 g 4-Piperidylcarbonsäure und 1,06 g Natriumcarbonat in 15 ml Dimethylformamid wird 2 Stunden bei 120°C gerührt. Die abgekühlte Suspension wird mit Wasser verdünnt. Man gibt 2,0 g Ammoniumchlorid zu und extrahiert die wässrige Phase mit Essigester. Die organische Phase wird mit gesättigter Kochsalzlösung gewaschen, über Natriumsulfat getrocknet und das Lösungsmittel unter vermindertem Druck entfernt.
Ausbeute: 1,2 g (52 % der Theorie),
$R_f$-Wert: 0,29 (Kieselgel; Methylenchlorid/Methanol/konz. Ammoniak = 4:1:0,25)

Beispiel VII

1-(5-Cyanopyrid-2-yl)-4-[4-(methoxycarbonylmethyl)-piperidinocarbonyl]-piperidin

2,3 g 4-Carboxy-1-(5-cyanopyrid-2-yl)-piperidin, 2,0 g 4-Piperidylessigsäure-methylester-hydrochlorid, 4,8 g 2-[(1H)-Benzotriazol-1-yl]-1,1,3,3-tetramethyl-uroniumtetrafluorobo rat und 5 ml Triethylamin in 75 ml Dimethylformamid wird 16 Stunden bei Raumtemperatur gerührt. Das Lösungsmittel wird unter vermindertem Druck entfernt. Der Rückstand wird mit Wasser verdünnt und die wässrige Phase mit Essigester extrahiert. Die organische Phase wird mit Wasser und gesättigter Kochsalzlösung gewaschen und über Natriumsulfat getrocknet. Das Lösungsmittel wird unter vermindertem Druck entfernt und der Rückstand über Kieselgel chromatographiert.
Ausbeute: 2,75 g (74 % der Theorie),
Schmelzpunkt: 112-115°C
$R_f$-Wert: 0,40 (Kieselgel; Methylenchlorid/Methanol = 15:1)
Analog Beispiel VII werden folgende Verbindungen erhalten:

(1) 1-(5-Cyanopyrid-2-yl)-4-[N-[trans-4-(methoxycarbonyl)-cyclohexyl]-aminocarbonyl)-piperidin
$R_f$-Wert: 0,39 (Kieselgel; Methylenchlorid/Methanol = 15:1)

(2) 1-(4-Cyanophenyl)-4-[N-(2-methoxycarbonylethyl)-aminocarbonylmethyl]-piperazin
Schmelzpunkt: 89-92°C

(3) 1-(4-Cyanophenyl)-4-[(3-methoxycarbonyl-propyl)-carbonylamino]-piperidin
Hergestellt aus 4-Amino-1-(4-cyano-phenyl)-piperidin und Glutarsäure-monomethylester.

(4) 1-(4-Cyanophenyl)-4-[(4-trans-methoxycarbonyl-cyclohexyl)-carbonylamino]-piperidin
Hergestellt aus 4-Amino-1-(4-cyano-phenyl)-piperidin und 1,4-trans-Cyclohexandicarbonsäure-monomethylester.

Beispiel VIII

4-(4-Carboxyphenyl)-piperidin-hydrochlorid

Zu einer Lösung von 63,0 g 1-Acetyl-4-phenyl-piperidin in 1000 ml Methylenchlorid tropft man unter gutem Rühren bei -10 bis -20 °C 157,4 g Oxalylchlorid, Anschließend gibt man 46,7 g Aluminiumchlorid zu. Man rührt 1 Stunde bei -10 °C und gibt weitere 82,7 g Aluminiumchlorid zu. Nach weiteren 2 Stunden wird das Kühlbad entfernt und 24 Stunden bei Raumtemperatur gerührt. Die Reaktionslösung wird vorsichtig in ca. 4 l Eis/Wasser eingerührt und die wässrige Phase zweimal mit Methylenchlorid extrahiert. Die vereinigten organischen Phasen werden mit Wasser gewaschen, über Natriumsulfat getrocknet, und das Lösungsmittel unter vermindertem Druck entfernt. Der verbleibende Rückstand wird unter kräftigem Rühren in 2,5 l 2N Natronlauge gelöst. Zur dunklen wässrigen Lösung gibt man Eis und säuert mit konz. Salzsäure an. Der Niederschlag wird abgenutscht, mit Wasser gewaschen und in 2 l 6N Salzsäure 5 Stunden zum Rückfluß erhitzt. Das Lösungsmittel wird unter vermindertem Druck entfernt. Der verbleibende Feststoff wird mit wenig Wasser verrieben und abgenutscht.
Ausbeute: 40,5 g (54 % der Theorie),
Schmelzpunkt: > 300 °C
$R_f$-Wert: 0,07 (Kieselgel; Methylenchlorid/Methanol/konz. Ammoniak = 4:1:0,25)

Beispiel IX

1-tert.Butyloxycarbonyl-4-(4-carboxyphenyl)-piperidin

Zu 16,4 g Natriumhydroxid in 300 ml Wasser gibt man vorsichtig 47,5 g 4-(4-Carboxyphenyl)-piperidin-hydrochlorid. Die Suspension wird mit 500 ml Dioxan und 250 ml Wasser verdünnt. Anschließend werden 54,6 g Pyrokohlensäure-di-tert.butylester portionsweise zugegeben. Man rührt 16 Stunden bei Raumtemperatur. Der Niederschlag wird abgenutscht und das Filtrat unter vermindertem Druck teilweise eingedampft. Der Niederschlag und das verbleibende wässrige Filtrat werden vereinigt und mit 1 l Wasser verdünnt. Die wässrige Phase wird mit gesättigter Kaliumhydrogensulfat-Lösung auf pH 2 gebracht und zweimal mit Essigester extrahiert. Die vereinigten Essigester-Phasen werden mit gesättigter Kochsalzlösung gewaschen, über Natriumsulfat getrocknet und das Lösungsmittel unter vermindertem Druck entfernt. Das kristalline Rohprodukt wird mit wenig Essigester verrieben, abgenutscht und getrocknet.
Ausbeute: 54,0 g (90 % der Theorie),
Schmelzpunkt: 172-174 °C
$R_f$-Wert: 0,73 (Kieselgel; Essigester/Cyclohexan = 4:1)

Beispiel X

4-(4-Aminocarbonylphenyl)-1-tert.butyloxycarbonyl-piperidin

Zu einer Lösung von 21,4 g 1-tert.Butyloxycarbonyl-4-(4-carboxyphenyl)-piperidin in 250 ml wasserfreiem Dimethylformamid gibt man bei -10 °C 9,5 g 1-Hydroxy-(1H)-benzotriazol und 17,3 g N,N'-Dicyclohexylcarbodiimid. Man rührt 15 Minuten bei -10 °C und läßt die Temperatur innerhalb einer Stunde auf Raumtemperatur ansteigen. Anschließend werden bei -10 °C 20 ml konz. Ammoniak unter kräftigem Rühren zugetropft. Es wird eine Stunde bei -10 °C und weitere 2 Stunden bei Raumtemperatur gerührt. Das Lösungsmittel wird unter vermindertem Druck entfernt. Der verbleibende Rückstand wird in Wasser suspendiert und die wässrige Phase viermal mit Essigester extrahiert. Die vereinigten Essigesterphasen werden filtriert, das Filtrat über Natriumsulfat getrocknet und das Lösungsmittel unter vermindertem Druck entfernt. Der verbleibende Rückstand wird über Kieselgel chromatographiert.
Ausbeute: 17,5 g (82 % der Theorie),
Schmelzpunkt: 188-191 °C
$R_f$-Wert: 0,36 (Kieselgel; Essigester/Cyclohexan = 4:1)

Beispiel XI

1-tert.Butyloxycarbonyl-4-(4-cyanophenyl)-piperidin

Eine Lösung von 4,5 g 4-(4-Aminocarbonylphenyl)-1-tert.butyloxycarbonyl-piperidin, 6,8 g Triphenylphosphin, 2,4 g Tetrachlorkohlenstoff und 1,6 g Triethylamin in 50 ml Chloroform wird 2 Stunden bei 60 °C gerührt. Es wird 1 ml Tetrachlorkohlenstoff zugetropft und weitere 3 Stunden bei 60 °C gerührt. Das Lösungsmittel wird unter vermindertem Druck entfernt und das verbleibende Öl über Kieselgel chromatographiert.
Ausbeute: 2,4 g (57 % der Theorie),
$R_f$-Wert: 0,32 (Kieselgel; Cyclohexan/Essigester = 4:1)

Beispiel XII

4-(4-Cyanophenyl)-piperidin-hydrochlorid

Eine Lösung von 2,4 g 1-tert.Butyloxycarbonyl-4-(4-cyanophenyl)-piperidin in 20 ml Dioxan und 20 ml etherischer Salzsäure wird 16 Stunden bei Raumtemperatur gerührt. Der kristalline Niederschlag wird abgenutscht, mit Ether gewaschen und getrocknet.
Ausbeute: 1,85 g (99 % der Theorie),
Schmelzpunkt: 284-288 °C
$R_f$-Wert: 0,79 (Kieselgel; Methylenchlorid/Methanol/konz. Ammoniak = 4:1:0,25)

Beispiel XIII

1-Methoxycarbonylmethyl-4-[(4-nitrophenyl)-oxycarbonyl]-piperidin

Zu einer Suspension von 2,91 g Piperid-4-yl-essigsäure-methylester-hydrochlorid und 3,02 g Chlorameisensäure-4-nitrophenylester in 150 ml Tetrahydrofuran tropft man bei 0 °C eine Lösung von 3,54 g Triethylamin in 10 ml Tetrahydrofuran. Es wird 3 Stunden bei 0 °C gerührt. Anschließend wird das Kühlbad entfernt und 16 Stunden bei Raumtemperatur gerührt. Der Niederschlag wird abgenutscht und das Filtrat unter vermindertem Druck entfernt. Der verbleibende Rückstand wird über Kieselgel chromatographiert. Das noch verunreinigte Produkt wird in Essigester gelöst und die organische Phase einmal mit 0,5N Natronlauge und einmal mit Wasser gewaschen. Die organische Phase wird über Natriumsulfat getrocknet und das Lösungsmittel unter vermindertem Druck entfernt.
Ausbeute: 3,30 g (68 % der Theorie),
Schmelzpunkt: 109-111 °C
$R_f$-Wert: 0,63 (Kieselgel; Essigester/Cyclohexan = 1:1)

Beispiel XIV

4-(4-Cyanophenyl)-1-[4-(methoxycarbonylmethyl)-piperidinocarbonyl]-piperidin

1,3 g 4-(4-Cyanophenyl)-piperidin und 2,9 g 4-(Methoxycarbonylmethyl)-1-[(4-nitrophenyl)-oxycarbonyl]-piperidin werden 2,5 Stunden bei 140 °C gerührt. Man läßt abkühlen und löst den Rückstand in Essigester. Die organische Phase wird zweimal mit 0,5N Natronlauge und einmal mit Wasser gewaschen. Die Essigester-Phase wird über Natriumsulfat getrocknet und das Lösungsmittel unter vermindertem Druck entfernt. Der verbleibende Rückstand wird über Kieselgel chromatographiert.
Ausbeute: 1,5 g (58 % der Theorie),
Schmelzpunkt: 115-118 °C
$R_f$-Wert: 0,32 (Kieselgel; Essigester/Cyclohexan = 1:1)
Analog Beispiel XIV werden folgende Verbindungen erhalten:

(1) 1-(4-Cyanophenyl)-4-[4-(methoxycarbonylmethyl)-piperidinocarbonyl]-piperazin
Schmelzpunkt: 99-100 °C

(2) 1-(4-Cyanophenyl)-4-[4-(ethoxycarbonylmethyliden)-piperidinocarbonyl]-piperazin (A) und 1-(4-Cyanophenyl)-4-[4-(ethoxycarbonylmethyl)-3,4-dehydro-piperidinocarbonyl]-piperazin (B) als Gemisch.
1-(4-Cyanophenyl)-piperazin wird mit einem Gemisch aus 1-[(4-Nitrophenyl)-oxycarbonyl]-4-(ethoxycar-

bonylmethyliden)-piperidin und 1-[(4-Nitrophenyl)-oxycarbonyl]-4-(ethoxycarbonylmethyliden)-3,4-dehydro-piperidin umgesetzt. Die anschließende Trennung erfolgt über Kieselgel.
$R_f$-Wert von (A): 0,55 (Kieselgel; Methylenchlorid/Essigester = 9:1)
$R_f$-Wert von (B): 0,42 (Kieselgel; Methylenchlorid/Essigester = 9:1)

Beispiel XV

1-(4-Cyanophenyl)-4-[N-[cis-4-(methoxycarbonyl)-cyclohexyl]-N-methyl-aminocarbonyl]-piperazin

Zu einer Suspension von 2,5 g cis-4-(Methylamino)-cyclohexancarbonsäuremethylester-hydrochlorid und 2,4 g Chlorameisensäure-p-nitrophenylester in 100 ml Tetrahydrofuran tropft man bei 0 °C eine Lösung von 4 ml Triethylamin in 20 ml Tetrahydrofuran. Man rührt 3 Stunden bei 0 °C und entfernt anschließend das Lösungsmittel unter vermindertem Druck. Der verbleibende Rückstand wird in Essigester gelöst und die organische Phase mit 1N Natronlauge, mit Wasser, mit 1N Salzsäure und mit gesättigter Kochsalzlösung gewaschen. Die organische Phase wird über Natriumsulfat getrocknet und das Lösungsmittel unter vermindertem Druck entfernt. Zu dem verbleibenden Rückstand gibt man 2,2 g 1-(4-Cyanophenyl)-piperazin und rührt 6 Stunden bei 140 °C. Der Rückstand wird in Essigester gelöst und die organische Phase mit 1N Natronlauge, mit gesättigter Kochsalzlösung, mit 1N Salzsäure und mit gesättigter Kochsalzlösung gewaschen. Die organische Phase wird über Natriumsulfat getrocknet und das Lösungsmittel unter vermindertem Druck entfernt. Das Rohprodukt wird in wenig siedendem Essigester gelöst. Nach der Zugabe von Cyclohexan läßt man abkühlen und nutscht den Niederschlag ab.
Ausbeute: 1,9 g (34 % der Theorie),
$R_f$-Wert: 0,70 (Kieselgel; Methylenchlorid/Methanol/kon. Ammoniak = 9:1:0,1)
Analog Beispiel XV wird folgende Verbindung erhalten:
(1) 1-(4-Cyanophenyl)-4-[N-benzyl-N-[trans-4-(methoxycarbonyl)-cyclohexyl]-aminocarbonyl]-piperazin
Das Rohprodukt wird über Kieselgel chromatographiert.
$R_f$-Wert: 0,61 (Kieselgel; Methylenchlorid/Methanol = 15:1)

Beispiel XVI

4-Carboxy-1-(4-cyanophenyl)-piperidin

1,2 g 4-Fluorbenzonitril und 1,07 g 4-Piperidylcarbonsäure werden in 20 ml Dimethylsulfoxid während 4 Stunden auf 150 °C erhitzt. Die dunkle Lösung wird nach dem Erkalten mit einer 0,5N Kaliumhydrogensulfat-Lösung verdünnt und die wässrige Phase mit Essigester erschöpfend extrahiert. Die vereinigten organischen Extrakte werden getrocknet und das Lösungsmittel unter vermindertem Druck entfernt. Der verbleibende Rückstand wird mit Äther verrieben, abgesaugt und getrocknet.
Ausbeute: 0,86 g (45 % der Theorie),
Schmelzpunkt: 230-234 °C (Zers.)
Analog Beispiel XVI wird folgende Verbindung erhalten:
(1) 1-(4-Cyano-phenyl)-4-tert.butyloxycarbonylamino-piperidin
Durch Umsetzung von 4-Fluorbenzonitril mit 4-tert.Butyloxycarbonylamino-piperidin

Beispiel XVII

1-(4-Cyanophenyl)-4-[4-(methoxycarbonylmethyl)-piperidinocarbonyl]-piperidin

Zu einer Lösung von 3,6 g 4-Carboxy-1-(4-cyanophenyl)-piperidin in 100 ml Tetrahydrofuran und 20 ml Dimethylformamid gibt man bei Raumtemperatur portionsweise 2,8 g N,N'-Carbonyldiimidazol und rührt eine Stunde. Anschließend gibt man 3,35 g 4-Piperidylessigsäuremethylester-hydrochlorid und 1,9 ml N-Methyl-morpholin zu und rührt 16 Stunden bei Raumtemperatur. Das Lösungsmittel wird unter vermindertem Druck entfernt und der Rückstand in Wasser und Methylenchlorid aufgenommen. Man säuert durch Zugabe von 2N Salzsäure bis zum pH 6 an, trennt die organische Phase ab und extrahiert die wässrige Phase zweimal mit Methylenchlorid. Die vereinigten Methylenchlorid-Phasen werden getrocknet und das Lösungsmittel unter vermindertem Druck entfernt. Der Rückstand wird mit Äther verrieben, abgesaugt und getrocknet.
Ausbeute: 4,1 g (71 % der Theorie),

Schmelzpunkt: 110-112 ° C
Analog Beispiel XVII werden folgende Verbindungen erhalten:
(1) 1-(4-Cyanophenyl)-4-[4-(ethoxycarbonylmethyliden)-piperidinocarbonyl]-piperidin
Unter Verwendung von 4-(Ethoxycarbonylmethyliden)-piperidin Öl
(2) 1-(4-Cyanophenyl)-4-[4-(ethoxycarbonylmethyl)-3,4-dehydropiperidinocarbonyl]-piperidin
Unter Verwendung von 4-(Ethoxycarbonylmethyl)-3,4-dehydropiperidin
Öl
(3) 1-(4-Cyanophenyl)-4-[N-[trans-4-(methoxycarbonyl)-cyclohexyl]-aminocarbonyl]-piperidin
Unter Verwendung von trans-4-Amino-cyclohexancarbonsäuremethylester
Schmelzpunkt: 230-232 ° C

Beispiel XVIII

1-(4-Cyanophenyl)-4-(methoxycarbonylmethyl)-piperazin

2 g 1-(4-Cyanophenyl)-piperazin werden in 20 ml trockenem Dimethylformamid bei Raumtemperatur mit 0,51 g NatriumhydridÖl-Suspension (50%ig) gerührt. Unter Kühlung im Wasserbad gibt man 1 ml Bromessigsäuremethylester zu. Anschließend wird mit Wasser verdünnt und mit Essigester extrahiert. Die vereinigten Essigester-Extrakte werden getrocknet und das Lösungsmittel unter vermindertem Druck entfernt. Der verbleibende Rückstand wird mit Methylenchlorid/Methanol (98:2) über Kieselgel chromatographiert. Das Produkt wird mit Äther verrieben, abgesaugt und mit Petroläther gewaschen.
Ausbeute: 1,6 g (58 % der Theorie),
Schmelzpunkt: 92-96 ° C

Beispiel XIX

1-(Carboxymethyl)-4-(4-cyanophenyl)-piperazin

Zu 3 g 1-(4-Cyanophenyl)-4-(methoxycarbonylmethyl)-piperazin in 46 ml Tetrahydrofuran gibt man bei Raumtemperatur und unter Rühren 58 ml einer 1 molaren wässrigen Lithiumhydroxid-Lösung und läßt die Reaktionslösung 3 Stunden bei Raumtemperatur stehen. Anschließend gibt man 3,2 g Ammoniumchlorid zu und destilliert das Tetrahydrofuran unter vermindertem Druck ab. Der Niederschlag wird abgenutscht, mit Wasser gewaschen und getrocknet.
Ausbeute: 2,2 g (77 % der Theorie),
Schmelzpunkt: über 300 ° C
$R_f$-Wert: 0,18 (Kieselgel; Methylenchlorid/Methanol = 9:1)

Beispiel XX

4-Hydroxymethyl-piperidin-hydrochlorid

25 g 4-Pyridylmethanol werden in 300 ml 50%iger Essigsäure in Gegenwart von 2 g Platindioxid bei Raumtemperatur und unter einem Wasserstoffdruck von 60 psi hydriert. Nach beendeter Wasserstoffaufnahme und Entfernung des Katalysators wird das Lösungsmittel unter vermindertem Druck entfernt. Der feste Rückstand wird in Äther aufgenommen und anschließend mit ätherischer Salzsäure das Hydrochlorid ausgefällt. Der Niederschlag wird abgesaugt und getrocknet.
Ausbeute: 28,3 g (82 % der Theorie),
Schmelzpunkt: 120-125 ° C

Beispiel XXI

1-(4-Cyanophenyl)-4-hydroxymethyl-piperidin

Eine Lösung aus 12,2 g 4-Hydroxymethyl-piperidin-hydrochlorid, 9,8 g 4-Fluorbenzonitril und 28,3 ml N-Ethyl-diisopropylamin wird 4 Stunden auf 140 ° C erhitzt. Nach dem Abkühlen wird mit Methylenchlorid und Methylenchlorid/Essigester (1:1) als Elutionsmittel über Kieselgel chromatographiert. Nach Entfernen des Lösungsmittels unter vermindertem Druck wird der verbleibende Rückstand mit Petroläther verrieben und abgesaugt.

Ausbeute: 5,1 g (29 % der Theorie),
Schmelzpunkt: 148-150 °C

Beispiel XXII

1-(4-Cyanophenyl)-4-mesyloxymethyl-piperidin

Zu einer Lösung von 5,5 g 1-(4-Cyanophenyl)-4-hydroxymethyl-piperidin und 5,16 g Triäthylamin in 150 ml Methylenchlorid tropft man langsam bei Raumtemperatur und unter Rühren 5,84 g Methansulfonsäurechlorid. Nach beendeter Zugabe läßt man über Nacht stehen und entfernt das Lösungsmittel unter vermindertem Druck. Der Rückstand wird mit Methylenchlorid/Methanol (50:1) über Kieselgel chromatographiert.
Ausbeute: 6,95 g (93 % der Theorie),
Massenspektrum: $(M+H)^+$ = 295

Beispiel XXIII

Gemisch aus 1-(4-Cyanophenyl)-4-iodmethyl-piperidin und 1-(4-Cyanophenyl)-1-aza-bicyclo[2.2.1]-heptanyliumiodid

Eine Lösung von 6,8 g 1-(4-Cyanophenyl)-4-mesyloxymethyl-piperidin und 17,4 g Natriumiodid in 100 ml Aceton wird 6 Stunden zum Rückfluß erhitzt. Das Lösungsmittel wird unter vermindertem Druck entfernt und der verbleibende Rückstand mit 300 ml Methylenchlorid zum Sieden erwärmt. Man filtriert und dampft das Filtrat unter vermindertem Druck zur Trockene ein. Der Rückstand wird aus Ethanol/Petrolether kristallisiert. (Eine Lösung dieses Kristallisats besteht aus einem Gemisch von 1-(4-Cyanophenyl)-4-iodmethyl-piperidin und 1-(4-Cyanophenyl)-1-aza-bicyclo[2.2.1]-heptanylium-iodid, wobei die quantitative Zusammensetzung von Temperatur, Konzentration und Lösungsmittel abhängig ist).
Ausbeute: 5,9 g (78 % der Theorie),
Massenspektrum: $M^+$ = 326

Beispiel XXIV

1-(4-Cyanophenyl)-4-[4-(methoxycarbonylmethyl)-piperidinomethyl]-piperidin

Eine Suspension aus 2,18 g 1-(4-Cyanophenyl)-4-iodmethyl-piperidin (Gemisch aus Beispiel XXV), 1,26 g 4-Piperidylessigsäuremethylester-hydrochlorid und 1,35 g Triäthylamin in 150 ml Dimethylformamid wird 24 Stunden auf 130 °C erhitzt. Nach dem Erkalten wird das Lösungsmittel unter vermindertem Druck entfernt und der Rückstand mit Methylenchlorid/Methanol (30:1) über Kieselgel chromatographiert. Das Lösungsmittel wird unter vermindertem Druck entfernt und das Rohprodukt ohne weitere Reinigung für die nächste Synthesestufe verwendet.
Analog werden folgende Verbindungen erhalten:
(1) 1-(4-Cyanophenyl)-4-[N-[trans-4-(methoxycarbonyl)-cyclohexyl]-aminomethyl]-piperidin
Durch Umsetzung mit trans-4-Amino-cyclohexancarbonsäure-methylester bei 60 °C.
(2) 1-(4-Cyanophenyl)-4-[N-[trans-4-(methoxycarbonyl)-cyclohexyl]-N-methyl-aminomethyl]-piperidin
Durch Umsetzung mit trans-4-Methylamino-cyclohexancarbonsäure-methylester.
(3) 1-(4-Cyanophenyl)-4-[4-(methoxycarbonylmethyliden)-piperidinomethyl]-piperidin
Durch Umsetzung mit 4-Methoxycarbonylmethyliden-piperidin bei 90 °C.

Beispiel XXV

N-[trans-4-(Methoxycarbonyl)-cyclohexyl]-N-(3-phenylpropyl)-amin-hydrochlorid

Eine Lösung von 2 g trans-4-Aminocyclohexancarbonsäure-methylester-hydrochlorid, 1,5 ml 3-Phenylpropionaldehyd und 1,3 g Natriumcyanborhydrid in 40 ml trockenem Methanol wird 20 Stunden bei Raumtemperatur gerührt. Man verdünnt mit Wasser und extrahiert mit Essigester. Die vereinigten Essigester-Phasen werden getrocknet, mit etherischer Salzsäure angesäuert und das Lösungsmittel unter vermindertem Druck entfernt. Der Rückstand wird mit Aceton verrieben, abgesaugt und mit Aceton und anschließend mit Äther gewaschen.

Ausbeute: 1,9 g (59,1 % der Theorie),
Schmelzpunkt: 258-260 °C

Beispiel XXVI

1-(4-Cyanophenyl)-4-[N-[trans-4-(methoxycarbonyl)-cyclohexyl]-N-(3-phenylpropyl)-aminocarbonyl]-piperidin

Eine Lösung von 1,37 g 4-Carboxy-1-(4-cyanophenyl)-piperidin, 1,85 g N-[trans-4-(Methoxycarbonyl)-cyclohexyl]-N-(3-phenylpropyl)-amin-hydrochlorid, 0,88 g 1-Hydroxy-(1H)-benzotriazol, 2,1 g 2-[(1H)-Benzotriazol-1-yl]-1,1,3,3-tetramethyluronium-tetrafluoroborat und 1,43 ml N-Methyl-morpholin ir 40 ml Dimethylformamid wird 6 Stunden auf 60 °C erwärmt. Nach dem Erkalten wird mit Natriumbicaronat-Lösung verdünnt und die wässsrige Phase mit Essigester extrahiert. Die vereinigten Essigester-Phasen werden mit verdünnter Zitronensäure-Lösung und anschließend mit einer verdünnten Natriumbicarbonat-Lösung gewaschen, getrocknet und das Lösungsmittel unter vermindertem Druck entfernt. Der Rückstand wird mit Methylenchlorid und Methylenchlorid/Methanol (8:2) über Kieselgel chromatographiert.
Ausbeute: 0,55 (19 % der Theorie),
Schmelzpunkt: 145-146 °C
Analog Beispiel XXVI werden folgende Verbindungen erhalten:
(1) 1-(4-Cyanophenyl)-4-[N-benzyl-N-[trans-4-(methoxycarbonyl)-cyclohexyl]-aminocarbonyl]-piperidin
(2) 1-(4-Cyanophenyl)-4-[N-[trans-4-(methoxycarbonyl)-cyclohexyl]-N-(2-phenylethyl)-aminocarbonyl]-piperidin
(3) 1-(4-Cyanophenyl)-4-[N-[trans-4-(methoxycarbonyl)-cyclohexyl]-N-(n-pentyl)-aminocarbonyl]-piperidin

Beispiel XXVII

1-[2-[4-(N-tert. Butyloxycarbonyl)-piperidino]-ethyl]-4-methoxycarbonyl-piperidin

Eine äquivalente Mischung von N-tert.Butyloxycarbonyl-4-(2-mesyloxy-ethyl)-piperidin, Piperidinocarbonsäuremethylester mit Triethylamin wird in Dimethylformamid 24 Stunden auf 130 °C erhitzt. Nach dem Erkalten wird das Lösungsmittel unter vermindertem Druck entfernt und der Rückstand in Methylenchlorid aufgeommen und mit Wasser gewaschen. Nach Trocknen über Natriumsulfat und Eindampfen wird der Rückstand mittels Methylenchalorid/Methanol als Elutionsmittel über Kieselgel chromatografiert. Das Rohprodukt wird ohne weitere Reinigung für die nächste Synthesestufe verwendet.

Beispiel XXVIII

4-Methoxycarbonyl-1-[2-(4-piperidino)-ethyl]-piperidin

Das oben gewonnene rohe 1-[2-(4-(N-tert.Butyloxycarbonyl)-piperidino)-ethyl]-4-methoxycarbonyl-piperidin wird in eine Mischung aus Methylenchlorid/Trifluoressigsäure (1:1) gelöst und 2 Stunden bei Raumtemperatur stehen gelassen. Anschließend wird im Vakuum zur Trockene eingeengt und der Rückstand zwischen Methylenchlorid und gesättigter wässeriger Natriumbicarbonat-Lösung verteilt. Die organische Phase wird abgetrennt, über Natriumsulfat getrocknet und im Vakuum zur Trockene eingeengt.

Analog Beispiel XXVIII wird folgende Verbindung erhalten:
(1) 4-Amino-1-(4-cyano-phenyl)-piperidin

Beispiel XXIX

1-(4-Cyanophenyl)-4-[2-(4-methoxycarbonyl-piperidino)-ethyl]-piperidin

Eine äquimolare Lösung von 4-Methoxycarbonyl-1-[2-(4-piperidino)-ethyl]-piperidin, 4-Fluorbenzonitril und N-Ethyl-diisopropylamin wird 4 Stunden auf 140 °C erhitzt. Nach dem Abkühlen wird mit Methylenchlorid/Methanol als Elutionsmittel über Kieselgel chromatographiert. Nach Entfernen des Lösungsmittels im Vakuum wird der verbleibende Rückstand mit Petrolether verrieben und abgesaugt.

Herstellung der Endprodukte:

Beispiel 1

1-(4-Amidinophenyl)-4-[N-[trans-4-(methoxycarbonyl)-cyclohexyl]-aminocarbonyl]-piperazin-hydrochlorid

Durch eine Lösung von 16,1 g 1-(4-Cyanophenyl)-4-[N-[trans-4-(methoxycarbonyl)-cyclohexyl]-aminocarbonyl]-piperazin in 250 ml absolutem Methanol leitet man bei Raumtemperatur 1,5 Stunden trockenen Chlorwasserstoff. Man rührt 3 Stunden bei Raumtemperatur und entfernt das Lösungsmittel bei einer Badtemperatur von 25-35 °C unter vermindertem Druck. Der Rückstand wird in 250 ml Methanol gelöst. Zur Lösung gibt man unter gutem Rühren langsam 50 g Ammoniumcarbonat und rührt 16 Stunden bei Raumtemperatur. Das Lösungsmittel wird unter vermindertem Druck entfernt und das Rohprodukt mit 200 ml Wasser verrieben und abgesaugt. Anschließend wird der Feststoff mit 300 ml heißem Aceton verrieben und erneut abgesaugt.
Ausbeute: 14,1 g (77 % der Theorie),
$R_f$-Wert: 0,21 (Kieselgel; Methylenchlorid/Methanol/konz. Ammoniak = 4:1:0,25)
Massenspektrum: $(M+H)^+$ = 388
Analog Beispiel 1 werden folgende Verbindungen erhalten:

(1) 1-(4-Amidinophenyl)-4-[N-[trans-4-(methoxycarbonyl)-cyclohexyl]-N-methyl-aminocarbonyl]-piperazin-hydrochlorid Das Rohprodukt wird über Kieselgel chromatographiert.
$R_f$-Wert: 0,27 (Kieselgel; Methylenchlorid/Methanol/konz. Ammoniak = 4:1:0,25)
Massenspektrum: $M^+$ = 401

(2) 1-(5-Amidinopyrid-2-yl)-4-[N-[trans-4-(methoxycarbonyl)-cyclohexyl]-aminocarbonyl]-piperazin-hydrochlorid
$R_f$-Wert: 0,21 (Kieselgel; Methylenchlorid/Methanol/konz. Ammoniak = 4:1:0,25)
Massenspektrum: $M^+$ = 388

(3) 1-(5-Amidinopyrid-2-yl)-4-[N-[trans-4-(methoxycarbonyl)-cyclohexyl]-aminocarbonyl]-piperazin-hydrochlorid
$R_f$-Wert: 0,15 (Kieselgel; Methylenchlorid/Methanol/konz. Ammoniak = 4:1:0,25)
Massenspektrum: $M^+$ = 387

(4) 1-(5-Cyanopyrid-2-yl)-4-[4-(methoxycarbonylmethyl)-piperidinocarbonyl]-piperidin-hydrochlorid
Das wasserlösliche Rohprodukt wird mit Methylenchlorid/Methanol verrieben und die Suspension über Kieselgel filtriert.
Das Filtrat wird unter vermindertem Druck eingedampft. Der Rückstand wird mit Aceton verrieben und abgesaugt.
$R_f$-Wert: 0,48 (Kieselgel; Methylenchlorid/Methanol/konz. Ammoniak = 9:1:0,1)
Massenspektrum: $M^+$ = 387

(5) 4-(4-Amidinophenyl)-1-[N-[trans-4-(methoxycarbonyl)-cyclohexyl]-aminocarbonyl]-piperidin-hydrochlorid
Das Rohprodukt wird über Kieselgel chromatographiert.
Schmelzpunkt: Sinterung ab 176 °C
$R_f$-Wert: 0,15 (Kieselgel; Methylenchlorid/Methanol/konz. Ammoniak = 4:1:0,25)
Massenspektrum: $(M+I)^+$ = 387

(6) 4-(4-Amidinophenyl)-1-[4-(methoxycarbonylmethyl)-piperidinocarbonyl]-piperidin-hydrochlorid
Das Rohprodukt wird über Kieselgel chromatographiert.
Schmelzpunkt: Sinterung ab 205 °C
$R_f$-Wert: 0,20 (Kieselgel; Methylenchlorid/Methanol/konz. Ammoniak = 4:1:0,25)
Massenspektrum: $M^+$ = 386

(7) 1-(4-Amidinophenyl)-4-[N-[cis-4-(methoxycarbonyl)-cyclohexyl]-N-methyl-aminocarbonyl]-piperazin-hydrochlorid
Das Rohprodukt wird über Kieselgel chromatographiert.
$R_f$-Wert: 0,27 (Kieselgel; Methylenchlorid/Methanol/konz. Ammoniak = 4:1:0,25)
Massenspektrum: $M^+$ = 401

(8) 1-(4-Amidinophenyl)-4-[N-benzyl-N-[trans-4-(methoxycarbonyl)-cyclohexyl]-aminocarbonyl]-piperazin-hydrochlorid
Das Rohprodukt wird über Kieselgel chromatographiert.
$R_f$-Wert: 0,26 (Kieselgel; Methylenchlorid/Methanol/konz. Ammoniak = 4:1:0,25)
Massenspektrum: $M^+$ = 477

(9) 1-(5-Amidinopyrid-2-yl)-4-[N-[trans-4-(methoxycarbonyl)-cyclohexyl]-N-methyl-aminocarbonyl]-piperazin-hydrochlorid
$R_f$-Wert: 0,45 (Reversed Phase Platte RP18; Methanol/5%ige Kochsalzlösung = 6:4)
Massenspektrum: $M^+$ = 402
(10) 4-(4-Amidinophenyl)-1-[N-[trans-4-(methoxycarbonyl)-cyclohexyl]-N-methyl-aminocarbonyl]-piperidin-hydrochlorid
$R_f$-Wert: 0,33 (Kieselgel; Methylenchlorid/Methanol/konz. Ammoniak = 4:1:0,25)
Massenspektrum: $(M+H)^+$ = 401
(11) 1-(5-Amidinopyrid-2-yl)-4-[N-benzyl-N-[trans-4-(methoxycarbonyl)-cyclohexyl]-aminocarbonyl]-piperazin-hydrochlorid
(12) 1-(4-Amidinophenyl)-4-[4-(methoxycarbonylmethyl)-piperidinocarbonyl]-piperidin-hydrochlorid
Schmelzpunkt: 228-232°C (Zers.)
(13) 1-(4-Amidinophenyl)-4-[N-(3-methoxycarbonylpropyl)-aminocarbonyl]-piperidin-dihydrochlorid
(14) 1-(4-Amidinophenyl)-4-[N-(3-methoxycarbonylpropyl)-N-methyl-aminocarbonyl]-piperidin-hydrochlorid
(15) 1-(4-Amidinophenyl)-4-[N-benzyl-N-(3-methoxycarbonylpropyl)-aminocarbonyl]-piperidin-hydrochlorid
(16) 1-(4-Amidinophenyl)-4-[N-(3-methoxycarbonylpropyl)-N-(2-phenylethyl)-aminocarbonyl]-piperidin-hydrochlorid
(17) 1-(4-Amidinophenyl)-4-[N-(3-methoxycarbonylpropyl)-N-(3-phenylpropyl)-aminocarbonyl]-piperidin-hydrochlorid
(18) 1-(4-Amidinophenyl)-4-[N-(3-methoxycarbonylpropyl)-N-(n-pentyl)-aminocarbonyl]-piperidin-hydrochlorid
(19) 1-(4-Amidinophenyl)-4-[4-(methoxycarbonylmethyliden)-piperidinocarbonyl]-piperidin-hydrochlorid
(20) 1-[4-Amidinophenyl]-4-[4-(methoxycarbonylmethyl)-3,4-dehydro-piperidinocarbonyl]-piperidin-hydrochlorid
Schmelzpunkt: 217-220°C (Zers.)
(21) 1-(4-Amidinophenyl)-4-[N-(2-methoxycarbonylethyl)-aminocarbonylmethyl]-piperidin-hydrochlorid
Schmelzpunkt: 240-242°C (Zers.)
(22) 1-(4-Amidinophenyl)-4-[N-(2-methoxycarbonylethyl)-N-methyl-aminocarbonylmethyl]-piperidin-hydrochlorid
(23) 1-(4-Amidinophenyl)-4-[N-(2-methoxycarbonylethyl)-N-(2-phenylethyl)-aminocarbonylmethyl]-piperidin-hydrochlorid
(24) 1-(4-Amidinophenyl)-4-[N-[trans-4-(methoxycarbonyl)-cyclohexyl]-aminocarbonyl]-piperidin-hydrochlorid
(25) 1-(4-Amidinophenyl)-4-[4-(methoxycarbonylmethyl)-piperidinocarbonyl]-piperazin-hydrochlorid
Schmelzpunkt: 248-251°C (Zers.)
(26) 1-(4-Amidinophenyl)-4-[4-(methoxycarbonylmethyliden)-piperidinocarbonyl]-piperazin-hydrochlorid
(27) 1-(4-Amidinophenyl)-4-[4-(methoxycarbonylmethyl)-3,4-dehydro-piperidinocarbonyl]-piperazin-hydrochlorid
Schmelzpunkt: 228-230°C (Zers.)
(28) 1-(4-Amidinophenyl)-4-[N-(2-methoxycarbonylethyl)-aminocarbonylmethyl]-piperazin-hydrochlorid
Schmelzpunkt: 228-230°C (Zers.)
(29) 1-(4-Amidinophenyl)-4-[N-(2-methoxycarbonylethyl)-N-(2-phenylethyl)-aminocarbonylmethyl]-piperazin-hydrochlorid
(30) 1-(5-Amidinopyrid-2-yl)-4-[N-[trans-4-(methoxycarbonyl)-cyclohexyl]-N-[(morpholinocarbonyl)-methyl]-aminocarbonyl]-piperazin-hydrochlorid
(31) 4-(4-Amidinophenyl)-1-[N-benzyl-N-[trans-4-(methoxycarbonyl)-cyclohexyl]-aminocarbonyl]-piperidin-hydrochlorid
(32) 4-(4-Amidinophenyl)-1-[N-[trans-4-(methoxycarbonyl)-cyclohexyl]-N-[(morpholinocarbonyl)-methyl]-aminocarbonyl]-piperidin-hydrochlorid
(33) 1-(4-Amidinophenyl)-4-[N-[4-(methoxycarbonyl)-butyl]-aminocarbonyl]-piperazin-hydrochlorid
(34) 4-(4-Amidinophenyl)-1-[N-[4-(methoxycarbonyl)-butyl]-N-methyl-aminocarbonyl]-piperidin-hydrochlorid
(35) 1-(5-Amidinopyrid-2-yl)-4-[N-[3-(methoxycarbonyl)-propyl]-aminocarbonyl]-piperidin-hydrochlorid
(36) 1-(4-Amidinophenyl)-4-[N-benzyl-N-[trans-4-(methoxycarbonyl)-cyclohexyl]-aminocarbonyl]-piperidin-hydrochlorid
(37) 1-(4-Amidinophenyl)-4-[N-[trans-4-(methoxycarbonyl)-cyclohexyl]-N-(2-phenylethyl)-aminocarbonyl]-piperidin-hydrochlorid

Schmelzpunkt: 288-290 °C (Zers.)

(38) 1-(4-Amidinophenyl)-4-[N-[trans-4-(methoxycarbonyl)-cyclohexyl]-N-(3-phenylpropyl)-aminocarbonyl]-piperidin-hydrochlorid
Schmelzpunkt: 242-245 °C (Zers.)

(39) 1-(4-Amidinophenyl)-4-[N-[trans-4-(methoxycarbonyl)-cyclohexyl]-N-(n-pentyl)-aminocarbonyl]-piperidin-hydrochlorid Schmelzpunkt: 270-272 °C (Zers.)

(40) 1-(4-Amidinophenyl)-4-[4-(methoxycarbonylmethyl)-piperidinomethyl]-piperidin-dihydrochlorid
Amorphe Substanz
Massenspektrum: $(M+H)^+$ = 373

(41) 1-(4-Amidinophenyl)-4-[N-[trans-4-(methoxycarbonyl)-cyclohexyl]-aminomethyl]-piperidin-dihydrochlorid

(42) 1-(4-Amidinophenyl)-4-[N-[trans-4-(methoxycarbonyl)-cyclohexyl]-N-methyl-aminomethyl]-piperidin-dihydrochlorid

(43) 1-(4-Amidinophenyl)-4-[4-(methoxycarbonylmethyliden)-piperidinomethyl]-piperidin-dihydrochlorid

(44) 4-(4-Amidinophenyl)-1-[N-[trans-4-(methoxycarbonyl)-cyclohexyl]-aminocarbonyl]-3,4-dehydro-piperidin-hydrochlorid

(45) 4-(4-Amidinophenyl)-1-[N-benzyl-N-[trans-4-(methoxycarbonyl)-cyclohexyl]-aminocarbonyl]-3,4-dehydro-piperidin-hydrochlorid

(46) 1-(4-Amidinophenyl)-4-[N-[trans-4-(methoxycarbonyl)-cyclohexyl]-aminocarbonyl]-2-piperidon-hydrochlorid

(47) 1-(4-Amidinophenyl)-4-[N-benzyl-N-[trans-4-(methoxycarbonyl)-cyclohexyl]-aminocarbonyl]-2-piperidon-hydrochlorid

(48) 1-(5-Amidinopyrimid-2-yl)-4-[N-[trans-4-(methoxycarbonyl)-cyclohexyl]-aminocarbonyl]-piperidin-hydrochlorid

(49) 1-(4-Amidino-2-methylphenyl)-4-[N-[trans-4-(methoxycarbonyl)-cyclohexyl]-aminocarbonyl]-piperazin-hydrochlorid

(50) 1-(5-Amidinopyrid-2-yl)-4-[4-(methoxycarbonylmethyl)-piperazinocarbonyl]-piperidin-hydrochlorid

(51) 1-(4-Amidinophenyl)-4-[2-[N-[2-(methoxycarbonyl)-ethyl]-amino]-ethyl]-piperidin-hydrochlorid

(52) 1-(4-Amidinophenyl)-4-[2-[4-(methoxycarbonyl)-piperidino]-ethyl]-piperidin-hydrochlorid

(53) 1-(4-Amidinophenyl)-4-[2-(4-methoxycarbonyl-piperidino)-ethyl]-piperidin

(54) 1-(4-Amidinophenyl)-4-[(3-methoxycarbonyl-propyl)-carbonylamino]-piperidin

(55) 1-(4-Amidinophenyl)-4-[(4-trans-methoxycarbonyl-cyclohexyl)-carbonylamino]-piperidin

(56) 1-(4-Amidinophenyl)-4-[(4-trans-methoxycarbonyl-cyclohexyl)-carbonylmethylamino]-piperidin

## Beispiel 2

1-(4-Amidinophenyl)-4-[N-[trans-4-carboxycyclohexyl]-aminocarbonyl]-piperazin-dihydrochlorid-hydrat

Eine Lösung von 4,0 g 1-(4-Amidinophenyl)-4-[N-[trans-4-(methoxycarbonyl)-cyclohexyl]-aminocarbonyl]-piperazin-hy drochlorid in 50 ml halbkonzentrierter Salzsäure wird 5 Stunden bei Raumtemperatur gerührt. Das Lösungsmittel wird unter vermindertem Druck entfernt, das Rohprodukt mit Aceton verrieben, abgenutscht und getrocknet.
Ausbeute: 4,2 g (96 % der Theorie),
$R_f$-Wert: 0,15 (Kieselgel; Methylenchlorid/Methanol/konz. Ammoniak = 2:1:0,25)

| | | | |
|---|---|---|---|
| Ber. x 2 HCl x 1 $H_2O$ | C 49,14 | H 6,73 | N 15,08 |
| Gef. | 49,49 | 6,86 | 14,78 |

Massenspektrum: $(M+H)^+$ = 374
Analog Beispiel 2 werden folgende Verbindungen erhalten:

(1) 1-(4-Amidinophenyl)-4-[N-(trans-4-carboxycyclohexyl)-N-methyl-aminocarbonyl]-piperazin-dihydrochlorid
Die Reaktionslösung wird 16 Stunden bei Raumtemperatur gerührt.
$R_f$-Wert: 0,30 (Kieselgel; Methylenchlorid/Methanol/konz. Ammoniak = 2:1:0,25)
Massenspektrum: $(M+H)^+$ = 388

(2) 1-(5-Amidinopyrid-2-yl)-4-[N-(trans-4-carboxycyclohexyl)-aminocarbonyl]-piperidin-dihydrochlorid
$R_f$-Wert: 0,63 (Reversed Phase Platte RP18; Methanol/5%ige Kochsalzlösung = 6:4)

Massenspektrum: $(M + H)^+$ = 374
(3) 4-(4-Amidinophenyl)-1-[N-(trans-4-carboxycyclohexyl)-aminocarbonyl]-piperidin-hydrochlorid
Das Produkt kristallisiert aus.
Schmelzpunkt: Sinterung ab 252 ° C
$R_f$-Wert: 0,13 (Kieselgel; Methylenchlorid/Methanol/konz. Ammoniak = 2:1:0,25)
Massenspektrum: $(M + H)^+$ = 373
(4) 4-(4-Amidinophenyl)-1-[4-(carboxymethyl)piperidinocarbonyl]-piperidin-hydrochlorid
$R_f$-Wert: 0,35 (Kieselgel; Methylenchlorid/Methanol/konz. Ammoniak = 2:1:0,25)
Massenspektrum: $(M + H)^+$ = 373
(5) 1-(4-Amidinophenyl)-4-[N-(cis-4-carboxycyclohexyl)-N-methyl-aminocarbonyl]-piperazin-dihydrochlorid
$R_f$-Wert: 0,50 (Kieselgel; Methylenchlorid/Methanol/konz. Ammoniak = 2:1:0,25)
Massenspektrum: $(M + H)^+$ = 388
(6) 1-(4-Amidinophenyl)-4-[N-benzyl-N-(trans-4-carboxycyclohexyl)-aminocarbonyl]-piperazin-dihydrochlorid
$R_f$-Wert: 0,47 (Kieselgel; Methylenchlorid/Methanol/konz. Ammoniak = 2:1:0,25)
Massenspektrum: $(M + H)^+$ = 464
(7) 1-(5-Amidinopyrid-2-yl)-4-[N-(trans-4-carboxycyclohexyl)-aminocarbonyl]-piperazin-dihydrochlorid
Schmelzpunkt: Sinterung ab 180 ° C
$R_f$-Wert: 0,18 (Kieselgel; Methylenchlorid/Methanol/konz. Ammoniak = 2:1:0,25)
Massenspektrum: $(M + H)^+$ = 375
(8) 1-(5-Amidinopyrid-2-yl)-4-[N-(trans-4-carboxycyclohexyl)-N-methyl-aminocarbonyl]-piperazin-dihydrochlorid
$R_f$-Wert: 0,63 (Reversed Phase Platte RP18; Methanol/5%ige Kochsalzlösung = 6:4)
Massenspektrum: $(M + H)^+$ = 389
(9) 1-(5-Amidinopyrid-2-yl)-4-[N-benzyl-N-(trans-4-carboxycyclohexyl)-aminocarbonyl]-piperazin-dihydrochlorid
(10) 1-(5-Aminomethylpyrid-2-yl)-4-[4-(carboxymethyl)-piperidinocarbonyl]-piperidin-dihydrochlorid
$R_f$-Wert: 0,09 (Kieselgel; Methylenchlorid/Methanol/konz. Ammoniak = 4:1:0,25)
Massenspektrum: $M^+$ = 360
(11) 4-(4-Aminomethylphenyl)-1-[N-(trans-4-carboxycyclohexyl)-aminocarbonyl]-piperidin-hydrochlorid
Es wird 20 Stunden bei Raumtemperatur gerührt. Das Rohprodukt wird mit wenig Methanol verrieben und abgenutscht.
Schmelzpunkt: Sinterung ab 243 ° C
$R_f$-Wert: 0,13 (Kieselgel; Methylenchlorid/Methanol/konz. Ammoniak = 4:1:0,25)
Massenspektrum: $(M-H)^-$ = 358
(12) 4-(4-Aminomethylphenyl)-1-[4-(carboxymethyl)-piperidinocarbonyl]-piperidin-hydrochlorid
$R_f$-Wert: 0,19 (Kieselgel; Methylenchlorid/Methanol/konz. Ammoniak = 4:1:0,25)
Massenspektrum: $(M + H)^+$ = 360
(13) 1-(4-Aminomethylphenyl)-4-[4-(carboxymethyl)-piperidinocarbonyl]-piperidin-dihydrochlorid
(14) 1-(4-Amidinophenyl)-4-[4-(carboxymethyliden)-piperidinocarbonyl]-piperidin-dihydrochlorid
(15) 1-(4-Amidinophenyl)-4-[4-(carboxymethyl)-3,4-dehydro-piperidinocarbonyl]-piperidin-dihydrochlorid
(16) 1-(4-Aminomethylphenyl)-4-[4-(carboxymethyliden)-piperidinocarbonyl]-piperidin-dihydrochlorid
(17) 1-(4-Amidinophenyl)-4-[4-(carboxymethyl)-piperidinocarbonyl]-piperazin-dihydrochlorid
Schmelzpunkt: 190-192 ° C (Zers.)
(18) 1-(4-Amidinophenyl)-4-[4-(carboxymethyliden)-piperidinocarbonyl]-piperazin-dihydrochlorid
(19) 1-(4-Amidinophenyl)-4-[4-(carboxymethyl)-3,4-dehydropiperidinocarbonyl]-piperazin-dihydrochlorid
Schmelzpunkt: 175-180 ° C (Zers.)
(20) 1-(4-Amidinophenyl)-4-[N-(2-carboxyethyl)-N-(2-phenylethyl)-aminocarbonylmethyl]-piperazin
(21) 1-(5-Amidinopyrid-2-yl)-4-[N-(trans-4-carboxycyclohexyl)-N-[(morpholinocarbonyl)-methyl]-aminocarbonyl]-piperazin-dihydrochlorid
(22) 1-(5-Aminomethylpyrid-2-yl)-4-[N-(trans-4-carboxycyclohexyl)-aminocarbonyl]-piperazin-dihydrochlorid
Schmelzpunkt: Sinterung ab 203 ° C
$R_f$-Wert: 0,17 (Kieselgel; Methylenchlorid/Methanol/konz. Ammoniak = 4:1:0,25)
Massenspektrum: $(M + H)^+$ = 362
(23) 4-(4-Amidinophenyl)-1-[N-benzyl-N-(trans-4-carboxycyclohexyl)-aminocarbonyl]-piperidin-hydrochlorid

(24) 4-(4-Amidinophenyl)-1-[N-(trans-4-carboxycyclohexyl)-N-[(morpholinocarbonyl)-methyl]-aminocarbonyl]-piperidin-hydrochlorid
(25) 1-(4-Amidinophenyl)-4-[N-(4-carboxybutyl)-aminocarbonyl]-piperazin-dihydrochlorid
(26) 4-(4-Amidinophenyl)-1-[N-(4-carboxybutyl)-N-methyl-aminocarbonyl]-piperidin-hydrochlorid
(27) 1-(5-Amidinopyrid-2-yl)-4-[N-(3-carboxypropyl)-aminocarbonyl]-piperidin-dihydrochlorid
(28) 1-(4-Amidinophenyl)-4-[N-(trans-4-carboxycyclohexyl)-aminomethyl]-piperidin-trihydrochlorid
(29) 1-(4-Aminomethylphenyl)-4-[N-(trans-4-carboxycyclohexyl)-aminomethyl]-piperidin-trihydrochlorid
(30) 1-(4-Amidinophenyl)-4-[N-(trans-4-carboxycyclohexyl)-N-methyl-aminomethyl]-piperidin-trihydrochlorid
(31) 1-(4-Aminomethylphenyl)-4-[N-(trans-4-carboxycyclohexyl)-N-methyl-aminomethyl]-piperidin-trihydrochlorid
(32) 1-(4-Aminomethylphenyl)-4-[4-(carboxymethyl)-piperidinomethyl]-piperidin-trihydrochlorid
(33) 4-(4-Amidinophenyl)-1-[N-(trans-4-carboxycyclohexyl)-aminocarbonyl]-3,4-dehydro-piperidin-hydrochlorid
(34) 4-(4-Amidinophenyl)-1-[N-benzyl-N-(trans-4-carboxycyclohexyl)-aminocarbonyl]-3,4-dehydro-piperidin-hydrochlorid
(35) 1-(4-Amidinophenyl)-4-[N-(trans-4-carboxycyclohexyl)-aminocarbonyl]-2-piperidon-hydrochlorid
(36) 1-(4-Amidinophenyl)-4-[N-benzyl-N-(trans-4-carboxycyclohexyl)-aminocarbonyl]-2-piperidon-hydrochlorid
(37) 1-(5-Amidinopyrimid-2-yl)-4-[N-(trans-4-carboxycyclohexyl)-aminocarbonyl]-piperidin-dihydrochlorid
(38) 1-(4-Amidino-2-methylphenyl)-4-[N-(trans-4-carboxycyclohexyl)-aminocarbonyl]-piperazin-dihydrochlorid
(39) 1-(5-Amidinopyrid-2-yl)-4-[4-(carboxymethyl)-piperazinocarbonyl]-piperidin-trihydrochlorid
(40) 1-(4-Amidinophenyl)-4-[2-[(2-carboxyethyl)-amino]-ethyl]-piperidin-trihydrochlorid
(41) 1-(4-Amidinophenyl)-4-[2-[4-carboxypiperidino]-ethyl]-piperidin-trihydrochlorid
(42) 1-(5-Amidinopyrid-2-yl)-4-[4-(carboxymethyl)-piperidinocarbonyl]-piperidin-dihydrochlorid
$R_f$-Wert: 0,12 (Kieselgel; Methylenchlorid/Methanol/konz. Ammoniak = 2:1:0,25)
Massenspektrum: $(M+H)^+$ = 374
(43) 1-(4-Amidinophenyl)-4-[4-(carboxymethyl)-piperidinomethyl]-piperidin-hydrochlorid
Schmelzpunkt: 255-257 °C
(44) 1-(4-Amidinophenyl)-4-[2-(4-carboxy-piperidino)-ethyl]-piperidin
(45) 1-(4-Amidinophenyl)-4-[(3-carboxy-propyl)-carbonylamino]-piperidin
(46) 1-(4-Amidinophenyl)-4-[(4-trans-carboxy-cyclohexyl)-carbonylamino]-piperidin
(47) 1-(4-Amidinophenyl)-4-[(4-trans-carboxy-cyclohexyl)-carbonylmethylamino]-piperidin

## Beispiel 3

### 1-(4-Aminomethylphenyl)-4-[N-(trans-4-carboxycyclohexyl)-aminocarbonyl]-piperazin

400 mg 1-(4-Aminomethylphenyl)-4-[N-[trans-4-(methoxycarbonyl)-cyclohexyl]-aminocarbonyl]-piperazin-hydrochlorid und 3 ml einer 1,4 molaren, wässrigen Lithiumhydroxid-Lösung in 3 ml Tetrahydrofuran und 2 ml Wasser wird 2 Stunden bei Raumtemperatur gerührt. Anschließend tropft man 3,2 ml 1N Salzsäure zu und dampft einen Teil des Lösungsmittels unter vermindertem Druck ab. Der Niederschlag wird abgenutscht, mit Aceton verrieben, erneut abgenutscht und getrocknet.
Ausbeute: 330 mg (94 % der Theorie),
$R_f$-Wert: 0,47 (Kieselgel; Methylenchlorid/Methanol/konz. Ammoniak = 2:1:0,25)
Massenspektrum: $M^+$ = 360
Analog Beispiel 3 werden folgende Verbindungen erhalten:
(1) 1-[4-(N-Benzyloxycarbonyl-amidino)-phenyl]-4-[N-(trans-4-carboxycyclohexyl)-aminocarbonyl]-piperazin
$R_f$-Wert: 0,41 (Kieselgel; Methylenchlorid/Methanol/konz. Ammoniak = 4:1:0,25)
Massenspektrum: $(M+H)^+$ = 508
(2) 4-(4-Amidinophenyl)-1-[N-(trans-4-carboxycyclohexyl)-aminocarbonyl]-piperidin
$R_f$-Wert: 0,42 (Kieselgel; Methylenchlorid/Methanol/konz. Ammoniak = 2:1:0,25)
(3) 1-(4-Aminomethylphenyl)-4-[N-(trans-4-carboxycyclohexyl)-N-methyl-aminocarbonyl]-piperazin
(4) 1-(4-Amidinophenyl)-4-[4-(carboxymethyl)-piperidinocarbonyl]-piperidin
Schmelzpunkt: 305-308 °C (Zers.)
(5) 1-[4-(N-Benzyloxycarbonyl-amidino)-phenyl]-4-[4-(carboxymethyl)-piperidinocarbonyl]-piperidin

(6) 1-(4-Amidinophenyl)-4-[N-(3-carboxypropyl)-aminocarbonyl]-piperidin
(7) 1-(4-Amidinophenyl)-4-[(3-carboxypropyl)-methylaminocarbonyl]-piperidin
(8) 1-(4-Amidinophenyl)-4-[N-benzyl-N-(3-carboxypropyl)-aminocarbonyl]-piperidin
(9) 1-(4-Amidinophenyl)-4-[N-(3-carboxypropyl)-N-(2-phenylethyl)-aminocarbonyl]-piperidin
(10) 1-(4-Amidinophenyl)-4-[N-(3-carboxypropyl)-N-(3-phenylpropyl)-aminocarbonyl]-piperidin
(11) 1-(4-Amidinophenyl)-4-[N-(3-carboxypropyl)-N-(n-pentyl)aminocarbonyl]-piperidin
(12) 1-(4-Amidinophenyl)-4-[N-(2-carboxyethyl)-aminocarbonylmethyl]-piperidin
(13) 1-(4-Aminomethylphenyl)-4-[N-(2-carboxyethyl)-N-methylaminocarbonylmethyl]-piperidin
(14) 1-(4-Amidinophenyl)-4-[N-(2-carboxyethyl)-N-(2-phenylethyl)-aminocarbonylmethyl]-piperidin
(15) 1-(4-Amidinophenyl)-4-[N-(trans-4-carboxycyclohexyl)-aminocarbonyl]-piperidin
(16) 1-(4-Amidinophenyl)-4-[N-(trans-4-carboxycyclohexyl)-N-methyl-aminocarbonyl]-piperidin
(17) 1-(4-Aminomethylphenyl)-4-[N-(trans-4-carboxycyclohexyl)-aminocarbonyl]-piperidin-hydrochlorid
Schmelzpunkt: 272-274 °C (Zers.)
(18) 1-(4-Aminomethylphenyl)-4-[4-(carboxymethyl)-piperidinocarbonyl]-piperazin
(19) 1-[4-(N-Allyloxycarbonyl-amidino)-phenyl]-4-[4-(carboxymethyl)-piperidinocarbonyl]-piperazin
(20) 1-(4-Amidinophenyl)-4-[N-(2-carboxyethyl)-aminocarbonylmethyl]-piperazin
Schmelzpunkt: 282-286 °C (Zers.)
(21) 4-[4-(N-Benzyloxycarbonyl-amidino)-phenyl]-1-[N-(trans-4-carboxycyclohexyl)-aminocarbonyl]-piperidin
(22) 1-(4-Amidinophenyl)-4-[4-(carboxymethyliden)-piperidinomethyl)-piperidin
(23) 1-(4-Amidinophenyl)-4-[N-benzyl-N-(trans-4-carboxycyclohexyl)-aminocarbonyl]-piperidin
(24) 1-(4-Amidinophenyl)-4-[N-(trans-4-carboxycyclohexyl)-N-(2-phenylethyl)-aminocarbonyl]-piperidin
Schmelzpunkt: 186-190 °C (Zers.)
(25) 1-(4-Amidinophenyl)-4-[N-(trans-4-carboxycyclohexyl)-N-(3-phenylpropyl)-aminocarbonyl]-piperidin
$R_f$-Wert: 0,66 (Kieselgel; Methylenchlorid/Methanol = 4:1)
(26) 1-(4-Amidinophenyl)-4-[N-(trans-4-carboxycyclohexyl)-N-(n-pentyl)-aminocarbonyl]-piperidin
Massenspektrum: $(M+H)^+$ = 443

Beispiel 4

1-(4-Aminomethylphenyl)-4-[N-[trans-4-(methoxycarbonyl)-cyclohexyl]-aminocarbonyl]-piperazin-hydrochlorid

Eine Lösung von 1,0 g 1-(4-Cyanophenyl)-4-[N-[trans-4-(methoxycarbonyl)-cyclohexyl]-aminocarbonyl]-piperazin in 100 ml Methanol und 15 ml etherischer Salzsäure wird in Gegenwart von 200 mg 10%igem Palladium auf Kohle 3 Stunden bei Raumtemperatur und einem Wasserstoffdruck von 3 bar hydriert. Anschließend wird der Katalysator abfiltriert und das Filtrat unter vermindertem Druck zur Trockne eingedampft. Das Rohprodukt wird in Methanol gelöst und anschließend mit Ether versetzt. Der Niederschlag wird abgenutscht und getrocknet.
Ausbeute: 790 mg (71 % der Theorie),
$R_f$-Wert: 0,62 (Kieselgel; Methylenchlorid/Methanol/konz. Ammoniak = 4:1:0,25)
Massenspektrum: $M^+$ = 374
Analog Beispiel 4 werden folgende Verbindungen erhalten:
(1) 1-(4-Aminomethylphenyl)-4-[N-[trans-4-(methoxycarbonyl)-cyclohexyl]-N-methyl-aminocarbonyl]-piperazin-hydrochlorid
(2) 1-(5-Aminomethylpyrid-2-yl)-4-[4-[(methoxycarbonyl)-methyl]-piperidinocarbonyl]-piperidin-dihydrochlorid
Die Hydrierung wird in Methanol/methanolischer Salzsäure (1:1) durchgeführt. Das Rohprodukt wird über Kieselgel chromatographiert. Das Produkt wird in methanolischer Salzsäure gelöst, das Lösungsmittel unter vermindertem Druck abgedampft, der Rückstand mit Ether verrieben und abgenutscht.
$R_f$-Wert: 0,67 (Kieselgel; Methylenchlorid/Methanol/konz. Ammoniak = 4:1:0,25)
Massenspektrum: $M^+$ = 374
(3) 4-(4-Aminomethylphenyl)-1-[N-[trans-4-(methoxycarbonyl)-cyclohexyl]-aminocarbonyl]-piperidin-hydrochlorid
Das Rohprodukt wird mit wenig Methanol verrieben und abgenutscht.
Schmelzpunkt: 238-242 °C
$R_f$-Wert: 0,44 (Kieselgel; Methylenchlorid/Methanol/konz. Ammoniak = 9:1:0,1)
Massenspektrum: $M^+$ = 373

(4) 4-(4-Aminomethylphenyl)-1-[4-[(methoxycarbonyl)-methyl]-piperidinocarbonyl]-piperidin-hydrochlorid
Die Hydrierung wird in Methanol/methanolischer Salzsäure (1:1) durchgeführt. Das Rohprodukt wird kristallin erhalten.
Schmelzpunkt: Sinterung ab 190 °C
$R_f$-Wert: 0,44 (Kieselgel; Methylenchlorid/Methanol/konz. Ammoniak = 9:1:0,1)
Massenspektrum: $M^+$ = 373
(5) 1-(4-Aminomethylphenyl)-4-[4-[(methoxycarbonyl)-methyl]-piperidinocarbonyl]-piperidin-dihydrochlorid
(6) 1-(4-Aminomethylphenyl)-4-[N-(3-carboxypropyl)-aminocarbonyl)-piperidin-dihydrochlorid
(7) 1-(4-Aminomethylphenyl)-4-[4-[(methoxycarbonyl)-methyl]-piperidinocarbonyl]-piperidin-dihydrochlorid
(8) 1-(4-Aminomethylphenyl)-4-[N-[2-(methoxycarbonyl)-ethyl]-N-methyl-aminocarbonylmethyl]-piperidin-dihydrochlorid
(9) 1-(4-Aminomethylphenyl)-4-[N-[trans-4-(methoxycarbonyl)-cyclohexyl]-aminocarbonyl]-piperidin-dihydrochlorid
Schmelzpunkt: 215-218 °C (Zers.)
(10) 1-(4-Aminomethylphenyl)-4-[4-(methoxycarbonylmethyl)-piperidinocarbonyl]-piperazin-dihydrochlorid
(11) 1-(5-Aminomethylpyrid-2-yl)-4-[N-[trans-4-(methoxycarbonyl)-cyclohexyl]-aminocarbonyl]-piperazin-dihydrochlorid Das Rohprodukt wird über Kieselgel chromatographiert.
$R_f$-Wert: 0,70 (Kieselgel; Methylenchlorid/Methanol/konz. Ammoniak = 4:1:0,25)
Massenspektrum: $M^+$ = 375
(12) 1-(4-Aminomethylphenyl)-4-[4-(methoxycarbonylmethyl)-piperidinomethyl]-piperidin-trihydrochlorid
(13) 1-(4-Aminomethylphenyl)-4-[N-[trans-4-(methoxycarbonyl)-cyclohexyl]-aminomethyl]-piperidin-trihydrochlorid
(14) 1-(4-Aminomethylphenyl)-4-[N-[trans-4-(methoxycarbonyl)-cyclohexyl]-N-methyl-aminomethyl]-piperidin-trihydrochlorid

## Beispiel 5

### 1-[4-(N-Methoxycarbonyl-amidino)-phenyl]-4-[N-[trans-4-(methoxycarbonyl)-cyclohexyl]-aminocarbonyl]-piperazin

Zu einer Suspension von 0,50 g 1-(4-Amidinophenyl)-4-[N-[trans-4-(methoxycarbonyl)-cyclohexyl]-aminocarbonyl]-piperazin-hydrochlorid in 30 ml Methylenchlorid tropft man 2,5 ml 1N Natronlauge und anschließend 0,10 ml Chlorameisensäuremethylester. Man rührt 40 Minuten bei Raumtemperatur. Die Lösung wird mit Wasser verdünnt und die wässrige Phase mit Methylenchlorid extrahiert. Die organische Phase wird mit gesättigter Kochsalzlösung gewaschen und über Natriumsulfat getrocknet. Das Lösungsmittel wird unter vermindertem Druck entfernt und der verbleibende Rückstand über Kieselgel chromatographiert.
Ausbeute: 0,36 g (69 % der Theorie),
$R_f$-Wert: 0,32 (Kieselgel; Methylenchlorid/Methanol = 15:1)
Massenspektrum: $(M+H)^+$ = 446
Analog Beispiel 5 werden folgende Verbindungen erhalten:
(1) 1-[4-(N-Benzyloxycarbonyl-amidino)-phenyl]-4-[N-[trans-4-(methoxycarbonyl)-cyclohexyl]-aminocarbonyl]-piperazin
Zu einer Suspension von 1-(4-Amidinophenyl)-4-[N-[trans-4-(methoxycarbonyl)-cyclohexyl]-aminocarbonyl]-piperazin-hydrochlorid und Chlorameisensäure-benzylester in Methylenchlorid/Wasser (5:1) tropft man solange 1N Natronlauge bis der pH-Wert bei 9 bleibt. Nach 2 Stunden Rühren bei Raumtemperatur wird das Produkt abgenutscht.
$R_f$-Wert: 0,86 (Kieselgel; Methylenchlorid/Methanol/konz. Ammoniak = 4:1:0,25)
Massenspektrum: $(M+H)^+$ = 522
(2) 1-[N-[trans-4-(methoxycarbonyl)-cyclohexyl]-aminocarbonyl]-4-[4-(N-ethoxycarbonyl-amidino)-phenyl]-piperazin
Unter Verwendung von Chlorameisensäure-ethylester
(3) 1-[4-(N-Methoxycarbonyl-amidino)-phenyl]-4-[N-[trans-4-(methoxycarbonyl)-cyclohexyl]-N-methyl-aminocarbony]-piperazin
(4) 1-[N-[trans-4-(methoxycarbonyl)-cyclohexyl]-N-methylaminocarbony]-4-[4-(N-ethoxycarbonyl-amidino)-phenyl]-piperazin
(5) 1-[4-(N-Methoxycarbonyl-amidino)-phenyl]-4-[4-(methoxycarbonylmethyl)-piperidinocarbonyl]-piperidin
Schmelzpunkt: 175-178 °C

(6) 1-[4-(N-Benzyloxycarbonyl-amidino)-phenyl]-4-[4-(methoxycarbonylmethyl)-piperidinocarbonyl]-piperidin
Unter Verwendung von Chlorameisensäure-benzylester
(7) 1-[4-(N-Acetoxymethoxycarbonyl-amidino)-phenyl]-4-[4-(methoxycarbonylmethyl)-piperidinocarbonyl]-piperidin
Unter Verwendung von Kohlensäure-acetyloxymethyl-(4-nitrophenyl)-ester und N-Ethyl-diisopropylamin.
(8) 1-[4-[N-(1-Acetoxy-ethoxycarbonyl)-amidino]-phenyl]-4-[4-(methoxycarbonylmethyl)-piperidinocarbonyl]-piperidin
Unter Verwendung von Kohlensäure-1-acetoxy-ethyl-(4-nitrophenyl)-ester und N-Ethyl-diisopropylamin
(9) 1-[4-(N-Methoxycarbonyl-amidino)-phenyl]-4-[N-(3-methoxycarbonylpropyl)-aminocarbonyl]-piperidin
(10) 1-[4-(N-Methoxycarbonyl-amidino)-phenyl]-4-[N-(3-methoxycarbonylpropyl)-N-methyl-aminocarbonyl]-piperidin
(11) 1-[4-(N-Benzyloxycarbonyl-amidino)-phenyl]-4-[4-(methoxycarbonylmethyliden)-piperidinocarbonyl]-piperidin
Unter Verwendung von Chlorameisensäure-benzylester.
(12) 1-[4-(N-Methoxycarbonyl-amidino)-phenyl]-4-[4-(methoxycarbonylmethyliden)-piperidinocarbonyl]-piperidin
(13) 1-[4-(N-Methoxycarbonyl-amidino)-phenyl]-4-[N-[trans-4-(methoxycarbonyl)-cyclohexyl]-aminocarbonyl]-piperidin
(14) 1-[4-[N-(1-Acetoxy-ethoxycarbonyl)-amidino]-phenyl]-4-[N-[trans-4-(methoxycarbonyl)-cyclohexyl]-N-methyl-aminocarbonyl]-piperidin
Unter Verwendung von Kohlensäure-1-acetoxyethyl-(4-nitrophenyl)-ester und Ethyl-diisopropylamin
(15) 1-[4-(N-Allyloxycarbonyl-amidino)-phenyl]-4-[4-(methoxycarbonylmethyl)-piperidinocarbonyl]-piperazin
Unter Verwendung von Chlorameisensäure-allylester und N-Ethyl-diisopropylamin
(16) 1-[4-(N-Methoxycarbonyl-amidino)-phenyl]-4-[N-(2-methoxycarbonylethyl)-aminocarbonylmethyl]-piperazin
(17) 1-[4-(N-Methoxycarbonyl-amidino)-phenyl]-4-[N-[trans-4-(isobutoxycarbonyl)-cyclohexyl]-aminocarbonyl]-piperazin
(18) 1-[4-(N-Methoxycarbonyl-amidino)-phenyl]-4-[N-[trans-4-(isopropoxycarbonyl)-cyclohexyl]-aminocarbonyl]-piperazin
(19) 1-[4-(N-Methoxycarbonyl-amidino)-phenyl]-4-[N-[trans-4-(isopropoxycarbonyl)-cyclohexyl]-N-methyl-aminocarbonyl]-piperazin
(20) 4-[4-(N-Benzyloxycarbonyl-amidino)-phenyl]-1-[N-[trans-4-(methoxycarbonyl)-cyclohexyl]-aminocarbonyl]-piperidin
Unter Verwendung von Chlorameisensäure-benzylester in Methylenchlorid/Wasser (5:1) und 1N Natronlauge.
(21) 4-[4-(N-Methoxycarbonyl-amidino)-phenyl]-1-[N-[trans-4-(methoxycarbonyl)-cyclohexyl]-aminocarbonyl]-piperidin
(22) 4-[4-(N-Methoxycarbonyl-amidino)-phenyl]-1-[N-[trans-4-(isopropoxycarbonyl)-cyclohexyl]-aminocarbonyl]-piperidin
(23) 1-[N-Benzyl-N-[trans-4-(methoxycarbonyl)-cyclohexyl]-aminocarbonyl]-4-[4-(N-methoxycarbonyl-amidino)-phenyl]-piperidin
(24) 1-[4-(N-Methoxycarbonyl-amidino)-phenyl]-4-[4-(methoxycarbonyl)-piperidinomethyl]-piperidin
(25) 1-[4-(N-Methoxycarbonyl-amidino)-phenyl]-4-[N-[trans-4-(methoxycarbonyl)-cyclohexyl]-aminocarbonyl]-piperidin
(26) 1-[4-(N-Methoxycarbonyl-amidino)-phenyl]-4-[2-(4-methoxycarbonyl-piperidino)-ethyl]-piperidin
(27) 1-[4-(N-Methoxycarbonyl-amidino)-phenyl]-4-[(3-methoxycarbonyl-propyl)-carbonylamino]-piperidin
(28) 1-[4-(N-Methoxycarbonyl-amidino)-phenyl]-4-[(4-transmethoxycarbonyl-cyclohexyl)-carbonylamino]-piperidin
(29) 1-[4-(N-Methoxycarbonyl-amidino)-phenyl]-4-[(4-transmethoxycarbonyl-cyclohexyl)-carbonylmethylamino]-piperidin

Beispiel 6

1-[4-(N-Benzyloxycarbonyl-amidino)-phenyl]-4-[N-[trans-4-[(morpholinocarbonyl)-methoxycarbonyl]-cyclohexyl]-aminocarbonyl]-piperazin

Zu einer Lösung von 1,5 g 1-[4-(N-Benzyloxycarbonyl-amidino)-phenyl]-4-[N-(trans-4-carboxycyclohexyl)-aminocarbonyl]-piperazin und 1,0 g Chloracetylmorpholid in 40 ml Dimethylsulfoxid gibt man 0,65 g Kaliumhydrogencarbonat und 0,9 g Kaliumcarbonat und rührt 2 Stunden bei Raumtemperatur. Die Reaktionslösung wird mit Wasser verdünnt und die wässrige Phase mit Methylenchlorid extrahiert. Die organische Phase wird mit gesättigter Kochsalzlösung gewaschen, über Aktivkohle filtriert und das Filtrat unter vermindertem Druck zur Trockne eingedampft. Der verbleibende Rückstand wird mit Ether verrieben und abgenutscht.
Ausbeute: 1,3 g (69 % der Theorie),
$R_f$-Wert: 0,38 (Kieselgel; Methylenchlorid/Methanol = 15:1)
Massenspektrum: $(M+H)^+$ = 635
Analog Beispiel 6 werden folgende Verbindungen erhalten:

(1) 1-[4-(N-Benzyloxycarbonyl-amidino)-phenyl]-4-[N-[trans-4-[(dimethylaminocarbonyl)-methoxycarbonyl]-cyclohexyl]-aminocarbonyl]-piperazin
Es wird α-Chlor-essigsäure-dimethylamid eingesetzt.
$R_f$-Wert: 0,40 (Kieselgel; Methylenchlorid/Methanol = 15:1)
Massenspektrum: $(M+H)^+$ = 593

(2) 1-[4-(N-Benzyloxycarbonyl-amidino)-phenyl]-4-[N-[trans-4-[(piperidinocarbonyl)-methoxycarbonyl]-cyclohexyl]-aminocarbonyl]-piperazin
Es wird α-Chlor-essigsäure-piperidid eingesetzt.

(3) 4-[4-(N-Benzyloxycarbonyl-amidino)-phenyl]-1-[N-[trans-4-[(dimethylaminocarbonyl)-methoxycarbonyl]-cyclohexyl]-aminocarbonyl]-piperidin

(4) 4-[4-(N-Benzyloxycarbonyl-amidino)-phenyl]-1-[N-[trans-4-[(piperidinocarbonyl)-methoxycarbonyl]-cyclohexyl]-aminocarbonyl]-piperidin

(5) 4-[4-(N-Benzyloxycarbonyl-amidino)-phenyl]-1-[N-[trans-4-[(morpholinocarbonyl)-methoxycarbonyl]-cyclohexyl]-aminocarbonyl]-piperidin

Beispiel 7

1-(4-Amidinophenyl)-4-[N-[trans-4-(isopropoxycarbonyl)-cyclohexyl]-aminocarbonyl]-piperazin-dihydrochlorid

Durch eine Suspension von 0,50 g 1-(4-Amidinophenyl)-4-[N-(trans-4-carboxycyclohexyl)-aminocarbonyl]-piperazin-dihydrochlorid in 60 ml Isopropanol leitet man bei 0 °C eine Stunde Chlorwasserstoff. Es wird 16 Stunden bei Raumtemperatur und 4 Stunden bei 60 °C gerührt. Das Lösungsmittel wird unter vermindertem Druck entfernt. Das Rohprodukt wird mit Aceton verrieben und abgenutscht.
Ausbeute: 0,40 g (73 % der Theorie),
$R_f$-Wert: 0,62 (Kieselgel; Methylenchlorid/Methanol7konz. Ammoniak = 2:1:0,25)
Massenspektrum: $M^+$ = 415
Analog Beispiel 7 werden folgende Verbindungen erhalten:

(1) 1-(4-Amidinophenyl)-4-[N-[trans-4-(cyclohexyloxycarbonyl)-cyclohexyl]-aminocarbonyl]-piperazin-dihydrochlorid
Die Umsetzung wird in einem Lösungsmittelgemisch aus Cyclohexan/Methylenchlorid durchgeführt. Nach dem Durchleiten von Chlorwasserstoff wird das Methylenchlorid abdestilliert und die Suspension 4 Stunden bei 90 °C gerührt. Das Produkt wird mit Ether gefällt.
$R_f$-Wert: 0,61 (Kieselgel; Methylenchlorid/Methanol/konz. Ammoniak = 2:1:0,25)
Massenspektrum: $(M+H)^+$ = 456

(2) 1-(4-Amidinophenyl)-4-[N-[trans-4-(cyclohexyloxycarbonyl)-cyclohexyl]-N-methyl-aminocarbonyl]-piperazin-dihydrochlorid
Die Umsetzung wird in einem Lösungsmittelgemisch aus Cyclohexanol/Methylenchlorid durchgeführt. Es wird 16 Stunden bei 50 °C gerührt.
$R_f$-Wert: 0,58 (Kieselgel; Methylenchlorid/Methanol/konz. Ammoniak = 2:1:0,25)
Massenspektrum: $(M+H)^+$ = 470

(3) 1-(4-Amidinophenyl)-4-[N-[trans-4-(isopropoxycarbonyl)-cyclohexyl]-N-methyl-aminocarbonyl]-piperazin-dihydrochlorid

Anstelle der freien Säure wird der Methylester eingesetzt. Es wird 24 Stunden bei 50 °C gerührt. Anschließend wird Eisessig zugegeben und weitere 24 Stunden bei 50 °C gerührt.
$R_f$-Wert: 0,21 (Reversed Phase Platte RP18; Methanol/5%ige Kochsalzlösung = 6:4)
Massenspektrum: $M^+$ = 429
(4) 1-(4-Amidinophenyl)-4-[N-[trans-4-(ethoxycarbonyl)-cyclohexyl]-N-methyl-aminocarbonyl]-piperazin-dihydrochlorid
(5) 1-(4-Amidinophenyl)-4-[N-[cis-4-(isopropoxycarbonyl)-cyclohexyl]-N-methyl-aminocarbonyl]-piperazin-dihydrochlorid
$R_f$-Wert: 0,27 (Reversed Phase Platte RP18; Methanol/5%ige Kochsalzlösung = 6:4)
Massenspektrum: $M^+$ = 429
(6) 1-(5-Amidinopyrid-2-yl)-4-[N-[trans-4-(isopropoxycarbonyl)-cyclohexyl]-N-methyl-aminocarbonyl]-piperazin-dihydrochlorid
$R_f$-Wert: 0,67 (Kieselgel; Methylenchlorid/Methanol/konz. Ammoniak = 2:1:0,25)
Massenspektrum: $(M+H)^+$ = 431
(7) 4-(4-Amidinophenyl)-1-[N-[trans-4-(isopropoxycarbonyl)-cyclohexyl]-aminocarbonyl]-piperidin-hydrochlorid
Die Umsetzung wird in Isopropanol/etherischer Salzsäure (5:2) durchgeführt. Es wird 24 Stunden bei Raumtemperatur gerührt. Das Rohprodukt wird mit Ether verrieben.
$R_f$-Wert: 0,27 (Kieselgel; Methylenchlorid/Methanol/konz. Ammoniak = 4:1:0,25)
Massenspektrum: $M^+$ = 414
(8) 4-(4-Amidinophenyl)-1-[N-[trans-4-(cyclohexyloxycarbonyl)-cyclohexyl]-aminocarbonyl]-piperidin-hydrochlorid
Die Umsetzung wird in Cyclohexanol/etherischer Salzsäure durchgeführt. Es wird 24 Stunden bei Raumtemperatur gerührt.
Das Rohprodukt wird mit Ether verrieben.
$R_f$-Wert: 0,25 (Kieselgel; Methylenchlorid/Methanol/konz. Ammoniak = 4:1:0,25)
Massenspektrum: $(M+H)^+$ = 455
(9) 4-(4-Amidinophenyl)-1-[N-[trans-4-(isobutoxycarbonyl)-cyclohexyl]-aminocarbonyl]-piperidin-hydrochlorid
Die Umsetzung wird in Isobutanol/etherischer Salzsäure (5:2) durchgeführt. Es wird 4 Stunden bei Raumtemperatur gerührt.
Das Rohprodukt wird mit Ether verrieben.
$R_f$-Wert: 0,24 (Kieselgel; Methylenchlorid/Methanol/konz. Ammoniak = 4:1:0,25)
Massenspektrum: $(M+H)^+$ = 429
(10) 4-(4-Amidinophenyl)-1-[N-methyl-N-[trans-4-(isopropoxycarbonyl)-cyclohexyl]-aminocarbonyl]-piperidin-hydrochlorid
Die Umsetzung wird in Isopropanol/etherischer Salzsäure (5:2) durchgeführt. Das Rohprodukt wird über Kieselgel chromatographiert.
$R_f$-Wert: 0,32 (Kieselgel; Methylenchlorid/Methanol/konz. Ammoniak = 4:1:0,25)
Massenspektrum: $(M+H)^+$ = 429
(11) 1-(4-Amidinophenyl)-4-[4-(ethoxycarbonylmethyl)-piperidinocarbonyl]-piperidin-dihydrochlorid
(12) 1-(4-Amidinophenyl)-4-[4-(isopropyloxycarbonylmethyl)-piperidinocarbonyl]-piperidin-dihydrochlorid
Schmelzpunkt: 208-210 °C (Zers.)
(13) 1-(4-Amidinophenyl)-4-[4-(cyclopentyloxycarbonylmethyl)-piperidinocarbonyl]-piperidin-dihydrochlorid
(14) 1-(4-Amidinophenyl)-4-[4-(cyclohexyloxycarbonylmethyl)-piperidinocarbonyl]-piperidin-dihydrochlorid
Schmelzpunkt: 250-252 °C (Zers.)
(15) 1-(4-Amidinophenyl)-4-[4-[(3-n-pentyloxy)-carbonylmethyl]-piperidinocarbonyl]-piperidin-dihydrochlorid
(16) 1-(4-Amidinophenyl)-4-[4-(isobutyloxy)-carbonylmethyl]-piperidinocarbonyl]-piperidin-dihydrochlorid
(17) 1-(4-Amidinophenyl)-4-[N-[3-(isopropyloxycarbonyl)-propyl]-N-methyl-aminocarbonyl]-piperidin-dihydrochlorid
(18) 1-(4-Amidinophenyl)-4-[4-(isopropoxycarbonylmethyliden)-piperidinocarbonyl]-piperidin-dihydrochlorid
(19) 1-(4-Amidinophenyl)-4-[N-[2-(benzyloxycarbonyl)-ethyl]-aminocarbonylmethyl]-piperidin-dihydrochlorid
(20) 1-(4-Amidinophenyl)-4-[N-[trans-4-(isopropyloxycarbonyl)-cyclohexyl]-aminocarbonyl]-piperidin-dihydrochlorid

Schmelzpunkt: 272-275 ° C (Zers.)

(21) 1-(4-Amidinophenyl)-4-[N-[trans-4-(isobutyloxycarbonyl)-cyclohexyl]-aminocarbonyl]-piperidin-dihydrochlorid

Schmelzpunkt: 282-284 ° C (Zers.)

(22) 1-(4-Amidinophenyl)-4-[N-[trans-4-(cyclohexyloxycarbonyl)-cyclohexyl]-aminocarbonyl]-piperidin-dihydrochlorid

Schmelzpunkt: 275-279 ° C (Zers.)

(23) 1-(4-Amidinophenyl)-4-[4-(isopropyloxycarbonylmethyl)-piperidinocarbonyl]-piperazin-dihydrochlorid

(24) 1-(4-Amidinophenyl)-4-[4-(isobutyloxycarbonylmethyl)-piperidinocarbonyl]-piperazin-dihydrochlorid

(25) 1-(4-Amidinophenyl)-4-[4-(cyclohexyloxycarbonylmethyl)-piperidinocarbonyl]-piperazin-dihydrochlorid

(26) 1-(4-Amidinophenyl)-4-[N-[2-(isopropyloxycarbonyl)-ethyl]-aminocarbonylmethyl]-piperazin-trihydrochlorid

(27) 1-(4-Amidinophenyl)-4-[N-[2-(3-n-pentyloxycarbonyl)-ethyl]-aminocarbonylmethyl]-piperazin-trihydrochlorid

(28) 1-(4-Amidinophenyl)-4-[N-[2-(cyclohexyloxycarbonyl)-ethyl]-aminocarbonylmethyl]-piperazin-trihydrochlorid

(29) 1-(4-Aminomethylphenyl)-4-[N-[trans-4-(isopropoxycarbonyl)-cyclohexyl]-aminocarbonyl]-piperazin-dihydrochlorid

(30) 1-(4-Amidinophenyl)-4-[N-[trans-4-(cyclopentyloxycarbonyl)-cyclohexyl]-N-methyl-aminocarbonyl]-piperazin-dihydrochlorid

(31) 1-(4-Aminomethylphenyl)-4-[N-[trans-4-(2-butyloxycarbonyl)-cyclohexyl]-N-methyl-aminocarbonyl]-piperazin-dihydrochlorid

(32) 1-(4-Amidinophenyl)-4-[N-benzyl-N-[trans-4-(isopropoxycarbonyl)-cyclohexyl]-aminocarbonyl]-piperazin-dihydrochlorid

(33) 1-(4-Amidinophenyl)-4-[N-benzyl-N-[trans-4-(cyclopentylmethoxycarbonyl)-cyclohexyl]-aminocarbonyl]-piperazin-dihydrochlorid

(34) 1-(5-Amidinopyrid-2-yl)-4-[N-[trans-4-(pyridylmethoxycarbonyl)-cyclohexyl]-aminocarbonyl]-piperazin-dihydrochlorid

(35) 1-(5-Amidinopyrid-2-yl)-4-[N-[trans-4-(isobutoxycarbonyl)-cyclohexyl]-aminocarbonyl]-piperazin-dihydrochlorid

(36) 1-(5-Aminomethylpyrid-2-yl)-4-[N-[trans-4-(cyclopentyloxycarbonyl)-cyclohexyl]-aminocarbonyl]-piperazin-dihydrochlorid

(37) 1-(5-Amidinopyrid-2-yl)-4-[N-[trans-4-(isopropoxycarbonyl)-cyclohexyl]-aminocarbonyl]-piperidin-dihydrochlorid

(38) 1-(5-Amidinopyrid-2-yl)-4-[N-[trans-4-(cyclohexylmethoxycarbonyl)-cyclohexyl]-aminocarbonyl]-piperidin-dihydrochlorid

(39) 4-(4-Amidinophenyl)-1-[N-[trans-4-(ethoxycarbonyl)-cyclohexyl]-aminocarbonyl]-piperidin-hydrochlorid

(40) 4-(4-Amidinophenyl)-1-[N-benzyl-N-[trans-4-(n-butyloxycarbonyl)-cyclohexyl]-aminocarbonyl]-piperidin-hydrochlorid

(41) 1-(4-Amidinophenyl)-4-[N-methyl-N-[trans-4-(isopropoxycarbonyl)-cyclohexyl]-aminomethyl]-piperidin-trihydrochlorid

(42) 1-(4-Amidinophenyl)-4-[N-[trans-4-(cyclohexyloxycarbonyl)-cyclohexyl]-N-methyl-aminomethyl]-piperidin-trihydrochlorid

(43) 1-(4-Amidinophenyl)-4-[4-[(isopropoxycarbonyl)-methyl]-piperidinomethyl]-piperidin-trihydrochlorid

(44) 1-(4-Amidinophenyl)-4-[4-[(cyclohexyloxycarbonyl)-methyl]-piperidinomethyl]-piperidin-trihydrochlorid

## Beispiel 8

### 1-(4-Amidinophenyl)-4-[N-[trans-4-(ethoxycarbonyl)-cyclohexyl]-aminocarbonyl]-piperazin-dihydrochlorid

Eine Suspension von 0,5 g 1-(4-Amidinophenyl)-4-[N-(trans-4-carboxycyclohexyl)-aminocarbonyl]-piperazin-dihydrochlorid in 10 ml Thionylchlorid wird 16 Stunden bei Raumtemperatur gerührt. Überschüssiges Thionylchlorid wird unter vermindertem Druck entfernt. Der verbleibende Rückstand wird in 20 ml Methylenchlorid und 20 ml Ethanol gelöst. Es wird 2 Stunden bei Raumtemperatur und 4 Stunden bei 50 ° C gerührt. Das Lösungsmittel wird unter vermindertem Druck entfernt und der verbleibende Rückstand mit Aceton verrieben und abgenutscht.

Ausbeute: 0,43 g (84 % der Theorie),

$R_f$-Wert: 0,61 (Kieselgel; Methylenchlorid/Methanol/konz. Ammoniak = 2:1:0,25)
Massenspektrum: $(M+H)^+$ = 402
Analog Beispiel 8 werden folgende Verbindungen erhalten:
(1) 1-(4-Amidinophenyl)-4-[4-[(2-(R)-butyloxy)-carbonylmethyl]-piperidinocarbonyl]-piperidin-dihydrochlorid
(2) 1-(4-Amidinophenyl)-4-[4-[(2-(S)-butyloxy)-carbonylmethyl]-piperidinocarbonyl]-piperidin-dihydrochlorid
(3) 4-(4-Amidinophenyl)-1-[4-[(2-(R)-butyloxy)-carbonylmethyl]-piperidinocarbonyl]-piperidin-hydrochlorid
(4) 4-(4-Amidinophenyl)-1-[4-[(2-(S)-butyloxy)-carbonylmethyl]-piperidinocarbonyl]-piperidin-hydrochlorid
(5) 1-(4-Amidinophenyl)-4-[4-[(2-morpholinoethyloxy)-carbonylmethyl]-piperidinocarbonyl]-piperidin-trihydrochlorid
(6) 1-(4-Amidinophenyl)-4-[4-[[2-(2-oxo-pyrrolidino)-ethyl]-oxycarbonylmethyl]-piperidinocarbonyl]-piperidin-dihydrochlorid
(7) 1-(4-Amidinophenyl)-4-[4-[(dimethylaminocarbonyl)-methoxycarbonylmethyl]-piperidinocarbonyl]-piperidin-dihydrochlorid
(8) 1-(4-Amidinophenyl)-4-[4-(exo-norborn-2-yloxycarbonylmethyl)-piperidinocarbonyl]-piperidin-dihydrochlorid
(9) 1-(4-Amidinophenyl)-4-[4-[trans-4-[(-)-menthyloxycarbonyl)-cyclohexyl]-aminocarbonyl]-piperidin-dihydrochlorid
(10) 1-(4-Amidinophenyl)-4-[4-[(2-(R)-butyloxy)-carbonylmethyl]-piperidinocarbonyl]-piperazin-dihydrochlorid
(11) 1-(4-Amidinophenyl)-4-[4-[(2-(S)-butyloxy)-carbonylmethyl]-piperidinocarbonyl]-piperazin-dihydrochlorid
(12) 1-(4-Amidinophenyl)-4-[N-[trans-4-(benzyloxycarbonyl)-cyclohexyl]-aminocarbonyl]-piperazin-dihydrochlorid
(13) 1-(4-Amidinophenyl)-4-[N-[trans-4-(cyclohexylmethoxycarbonyl)-cyclohexyl]-aminocarbonyl]-piperazin-dihydrochlorid
(14) 1-(4-Amidinophenyl)-4-[N-[trans-4-(isobutoxycarbonyl)-cyclohexyl]-aminocarbonyl]-piperazin-dihydrochlorid
(15) 1-(4-Amidinophenyl)-4-[N-[trans-4-(2-morpholino-ethoxycarbonyl)-cyclohexyl]-aminocarbonyl]-piperazin-dihydrochlorid
(16) 1-(4-Amidinophenyl)-4-[N-[trans-4-(endo-norborn-2-yloxycarbonyl)-cyclohexyl]-aminocarbonyl]-piperazin-dihydrochlorid

## Beispiel 9

### 1-(4-Amidinophenyl)-4-[N-[trans-4-[(morpholinocarbonyl)-methoxycarbonyl]-cyclohexyl]-aminocarbonyl]-piperazin-hydrochlorid

Eine Lösung von 300 mg 1-[4-(N-Benzyloxycarbonyl-amidino)-phenyl]-4-[N-[trans-4-[-(morpholinocarbonyl)-methoxycarbonyl]-cyclohexyl]-aminocarbonyl]-piperazin in 20 ml Dimethylformamid wird 2 Stunden in Gegenwart von 0,3 g eines Katalysators von 10 % Palladium auf Aktivkohle und einem Wasserstoff-Überdruck von 5 bar hydriert. Man gibt etherische Salzsäure zu und filtriert. Das Filtrat wird unter vermindertem Druck eingedampft und der verbleibende Rückstand jeweils mit Ether/Aceton, Aceton und Aceton/Methylenchlorid verrieben und abgenutscht.
Ausbeute: 210 mg (83 % der Theorie),
$R_f$-Wert: 0,27 (Kieselgel; Methylenchlorid/Methanol/konz. Ammoniak = 4:1:0,25)
Massenspektrum: $(M+H)^+$ = 501
Analog Beispiel 9 werden folgende Verbindungen erhalten:
(1) 1-(4-Amidinophenyl)-4-[N-[trans-4-[(dimethylaminocarbonyl)-methoxycarbonyl]-cyclohexyl]-aminocarbonyl]-piperazin-hydrochlorid
Die Hydrierung wird in einem Lösungsmittelgemisch aus Aceton/Dimethylformamid durchgeführt. Der Katalysator wird abfiltriert und das Rohprodukt mit Ether gefällt. Anschließend wird mit etherischer Salzsäure das Hydrochlorid hergestellt.
$R_f$-Wert: 0,15 (Kieselgel; Methylenchlorid/Methanol/konz. Ammoniak = 4:1:0,25)
Massenspektrum: $(M+H)^+$ = 459
(2) 1-(4-Amidinophenyl)-4-[N-[trans-4-[(piperidinocarbonyl)-methoxycarbonyl]-cyclohexyl]-aminocarbonyl]-piperazin-hydrochlorid

(3) 4-(4-Amidinophenyl)-1-[N-[trans-4-[(dimethylaminocarbonyl)-methoxycarbonyl]-cyclohexyl]-aminocarbonyl]-piperidin-hydrochlorid
(4) 4-(4-Amidinophenyl)-1-[N-[trans-4-[(piperidinocarbonyl)-methoxycarbonyl]-cyclohexyl]-aminocarbonyl]-piperidin-hydrochlorid
(5) 4-(4-Amidinophenyl)-1-[N-[trans-4-[(morpholinocarbonyl)-methoxycarbonyl]-cyclohexyl]-aminocarbonyl]-piperidin-hydrochlorid

Beispiel 10

1-[4-(N-Benzyloxycarbonylamidino)-phenyl]-4-[4-[(pivaloyloxymethyloxycarbonylmethyl]-piperidinocarbonyl]-piperidin

Eine Suspension von 1 Equivalent 1-[4-(N-Benzyloxycarbonylamidino)-phenyl]-4-[4-(carboxymethyl)-piperidinocarbonyl]-piperidin wird mit jeweils 2 Equivalenten Pivalinsäure-chlormethylester, Kaliumjodid, Kaliumbicarbonat und Kaliumcarbonat in Dimethylformamid bei Raumtemperatur 2 Tage gerührt. Nach Eingießen in Wasser, Extraktion mit Essigester und Eindampfen des Essigester-Extrakts erhält man das gewünschte Produkt, welches über Kieselgel chromatographiert wird.

Analog Beispiel 10 werden folgende Verbindungen erhalten:

(1) 1-[4-(N-Benzyloxycarbonyl-amidino)-phenyl]-4-[4-[[1-(ethoxycarbonyloxy)-ethoxycarbonyl]-methyl]-piperidinocarbonyl]-piperidin
Unter Verwendung von 1-(Ethoxycarbonyloxy)-ethylchlorid
(2) 1-[4-(N-Benzyloxycarbonyl-amidino)-phenyl]-4-[trans-4-(pivaloyloxymethyloxycarbonyl)-cyclohexylaminocarbonyl]-piperazin
Massenspektrum: $(M+H)^+$ = 622
(3) 1-[4-(N-Benzyloxycarbonyl-amidino)-phenyl]-4-[[trans-4-[1-(ethoxycarbonyloxy)-ethoxycarbonyl]-cyclohexylamino]-carbonyl]-piperazin
Unter Verwendung von 1-(Ethoxycarbonyloxy)-ethylchlorid
Massenspektrum: $(M+H)^+$ = 624

Beispiel 11

1-(4-Amidinophenyl)-4-[4-[(pivaloyloxymethyl)-oxycarbonylmethyl]-piperidinocarbonyl]-piperidin-hydrochlorid

1-[4-(N-Benzyloxycarbonyl-amidino)-phenyl]-4-[4-[(pivaloyloxymethyl)-oxycarbonylmethyl]-piperidinocarbonyl]-piperidin wird in Dimethylformamid/0,5N Salzsäure (10:1) unter Verwendung von Palladium auf Kohle (10%ig) unter einem Druck von 5 bar hydriert. Nach Entfernen des Katalysators wird ein pH-Wert von 3 eingestellt und das Lösungsmittel unter vermindertem Druck entfernt. Der verbleibende Rückstand wird in Aceton aufgeschlämmt und abgesaugt.

Analog Beispiel 11 werden folgende Verbindungen erhalten:

(1) 1-(4-Amidinophenyl)-[4-[1-(ethoxycarbonyloxy)-ethoxycarbonylmethyl]-piperidinocarbonyl]-piperidin-hydrochlorid
(2) 1-(4-Amidinophenyl)-4-[trans-4-(pivaloyloxymethyloxycarbonyl)-cyclohexylaminocarbonyl]-piperazin-hydrochlorid
Massenspektrum: $(M+H)^+$ = 488
(3) 1-(4-Amidinophenyl)-4-[[trans-4-[1-(ethoxycarbonyloxy)-ethoxycarbonyl]-cyclohexylamino]-carbonyl]-piperazin-hydrochlorid
Massenspektrum: $(M+H)^+$ = 490

Beispiel 12

Trockenampulle mit 2,5 mg Wirkstoff pro 1 ml

| Zusammensetzung: | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 13

Trockenampulle mit 35 mg Wirkstoff pro 2 ml

| Zusammensetzung: | |
|---|---|
| Wirkstoff | 35,0 mg |
| Mannitol | 100,0 mg |
| Wasser für Injektionszwecke ad | 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 14

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 15

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 16

Kapseln mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 17

Kapseln mit 350 mg Wirkstoff

| Zusammensetzung: | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Carbonsäurederivate der allgemeinen Formel

A - B - C - D - E - F - G ,(I)

in der
A eine geradkettige oder verzweigte Aminoalkylgruppe, eine Amino-, Amidino- oder Guanidinogruppe, wobei in jeder der vorstehend erwähnten Gruppen jeweils an einem der Stickstoffatome ein oder zwei Wasserstoffatome durch eine Alkyl- oder Phenylalkylgruppe oder ein Wasserstoffatom durch eine Alkoxycarbonylgruppe mit insgesamt 2 bis 5 Kohlenstoffatomen, durch eine $R_1$-CO-O-($R_2$cH)-O-CO-, Benzyloxycarbonyl- oder Allyloxycarbonylgruppe ersetzt sein kann, wobei

$R_1$ eine Alkylgruppe mit 1 bis 5 Kohlenstoffatomen, eine Cycloalkylgruppe mit 5 bis 7 Kohlenstoffatomen, eine Phenyl- oder Phenylalkylgruppe und

$R_2$ ein Wasserstoffatom, eine Alkylgruppe mit 1 bis 4 Kohlenstoffatomen, eine Cyloalkylgruppe mit 5 bis 7 Kohlenstoffatomen oder eine Phenylgruppe darstellen,

B einen 6-gliedrigen Aromaten, der ein oder zwei Stickstoffatome enthalten und zusätzlich im Kohlenstoffgerüst durch eine Trifluormethylgruppe monosubstituiert oder durch Fluor-, Chlor- oder Bromatome oder Alkylgruppen, wobei die Substituenten gleich oder verschieden sein können, mono- oder disubstituiert sein kann,

C eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sein können, oder eine 1,4-Cyclohexylengruppe, in der die Methylengruppe in 2-Stellung oder die Methylengruppen in 2- und 5-Stellung jeweils durch eine -$NR_3$-Gruppe ersetzt sind, wobei in einem so erhaltenen Ring, der ein oder zwei Stickstoffatome oder eine oder zwei -$NR_3$-Gruppen enthält, jeweils eine zu einem Stickstoffatom benachbarte Methylengruppe durch eine Carbonylgruppe ersetzt sein kann, oder eine 3,4-Dehydro-1,4-piperidinylengruppe, wobei

$R_3$ ein Wasserstoffatom, eine Alkyl- oder Phenylalkylgruppe darstellt,

D eine Methylen-, Ethylen-, Carbonyl- oder Methylencarbonylgruppe, wobei die Carbonylgruppe der Methylencarbonylgruppe mit dem Rest E verknüpft ist, oder eine -$NR_3$-CO-X-Gruppe, in der

das Stickstoffatom der -$NR_3$-CO-X-Gruppe mit dem Rest C verknüpft ist, $R_3$ wie vorstehend erwähnt definiert ist und

X eine geradkettige oder verzweigte Alkylengruppe mit 1 bis 5 Kohlenstoffatomen oder eine 1,4-Cyclohexylengruppe darstellt,

E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch ein Stickstoffatom oder durch eine >C=CH-Gruppe ersetzt sein kann, oder eine -$NR_4$-X-Gruppe, in der

X wie vorstehend erwähnt definiert ist und

$R_4$ ein Wasserstoffatom, eine Alkylgruppe mit 1 bis 6 Kohlenstoffatomen, welche in 1-, 2- oder 3-Stellung durch eine Morpholinocarbonyl-, Pyrrolidinocarbonyl-, Piperidinocarbonyl-oder Hexamethyleniminocarbonylgruppe oder durch eine ($R_1NR_2$)-CO-Gruppe substituiert sein kann, wobei $R_1$ und $R_2$ wie vorstehend erwähnt definiert sind, oder eine Phenylalkylgruppe mit 1 bis 4 Kohlenstoffatomen im Alkylteil darstellen,

F eine Alkylengruppe oder auch, falls E keine 1,4-Piperazinylengruppe darstellt, eine Bindung und

G eine durch eine $R_5$O-Gruppe substituierte Carbonylgruppe, in der

$R_5$ ein Wasserstoffatom, eine Alkylgruppe mit 1 bis 6 Kohlenstoffatomen, die in 1-, 2-, 3-Stellung durch eine Morpholinocarbonyl-, Pyrrolidinocarbonyl-, Piperidinocarbonyl-, Hexamethyleniminocarbonyl- oder ($R_1NR_2$)-CO-Gruppe oder in 2- oder 3-Stellung durch eine Morpholino- oder Pyrrolidinon-1-yl-Gruppe substituiert sein kann, wobei $R_1$ und $R_2$ wie vorstehend erwähnt definiert sind, eine Phenylalkyl- oder Pyridylalkylgruppe, eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe mit 1 bis 4 Kohlenstoffatomen, durch eine Alkylgruppe mit 1 bis 4 Kohlenstoffatomen und durch 1 bis 3 Methylgruppen substituiert sein kann, eine Bicycloalkyl- oder Bicycloalkylalkylgruppe, in denen der Bicycloalkylteil jeweils 6 bis 10 Kohlenstoffatome enthält und zusätzlich durch 1 bis 3 Methylgruppen substituiert sein kann, darstellt,
eine Phosphono-, O-Alkylphosphono-, Tetrazol-5-yl- oder $R_6$CO-O-CH$R_2$-O-CO-Gruppe darstellen, wobei
$R_2$ wie vorstehend erwähnt definiert ist und
$R_6$ eine Alkylgruppe mit 1 bis 5 Kohlenstoffatomen, eine Cycloalkyl- oder Cycloalkoxygruppe mit jeweils 5 bis 7 Kohlenstoffatomen im Cycloalkylteil, eine Alkoxygruppe mit 1 bis 4 Kohlenstoffatomen, eine Phenyl-, Phenylalkyl- oder Phenylalkoxygruppe darstellt,
wobei, soweit nichts anderes erwähnt wurde,
die vorstehend erwähnten Alkyl-, Alkylen- oder Alkoxyteile jeweils 1 bis 3 Kohlenstoffatome enthalten können.
deren Tautomere, deren Stereoisomere einschließlich deren Gemische und deren Additionssalze.

**2.** Carbonsäurederivate der allgemeinen Formel I gemäß Anspruch 1, in der
A eine Aminoalkylgruppe mit 1 oder 2 Kohlenstoffatomen oder eine Amidinogruppe, wobei in den vorstehend erwähnten Gruppen an einem Stickstoffatom ein Wasserstoffatom durch eine Alkoxycarbonylgruppe mit insgesamt 2 oder 3 Kohlenstoffatomen, durch eine Benzyloxycarbonyl-, Allyloxycarbonyl- oder $R_1$-CO-O-($R_2$CH)-O-CO-Gruppe ersetzt sein kann, wobei
$R_1$ eine Alkylgruppe mit 1 oder 2 Kohlenstoffatomen und
$R_2$ ein Wasserstoffatom oder eine Alkylgruppe mit 1 oder 2 Kohlenstoffatomen darstellen,
B einen 6-gliedrigen Aromaten, der ein oder zwei Stickstoffatome enthalten und zusätzlich im Kohlenstoffgerüst durch ein Fluor-, Chlor- oder Bromatom oder durch eine Methylgruppe substituiert sein kann,
C eine 3,4-Dehydro-1,4-piperidinylengruppe, eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sein können, oder eine 2-Oxo-1,4-piperidinylengruppe,
D eine Methylen-, Ethylen-, Carbonyl- oder Methylencarbonylgruppe, wobei die Carbonylgruppe der Methylencarbonylgruppe mit dem Rest E verknüpft ist, oder eine -N$R_3$-CO-X-Gruppe, in der
das Stickstoffatom der -N$R_3$-CO-X-Gruppe mit dem Rest C verknüpft ist,
$R_3$ ein Wasserstoffatom, eine Alkyl- oder Phenylalkylgruppe mit jeweils 1 bis 3 Kohlenstoffatomen im Alkylteil und
X eine geradkettige oder verzweigte Alkylengruppe mit 1 bis 4 Kohlenstoffatomen oder eine 1,4-Cyclohexylengruppe darstellen,
E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch ein Stickstoffatom oder durch eine >C = CH-Gruppe ersetzt sein kann, oder eine -N$R_4$-X-Gruppe, in der
X wie vorstehend erwähnt definiert ist und
$R_4$ ein Wasserstoffatom, eine Alkylgruppe mit 1 bis 6 Kohlenstoffatomen, eine Phenylalkylgruppe mit 1 bis 4 Kohlenstoffatomen im Alkylteil oder eine Morpholinocarbonylmethylgruppe darstellen,
F eine Methylengruppe oder auch, falls E keine 1,4-Piperazinylengruppe darstellt, eine Bindung und
G eine durch eine $R_5$O-Gruppe substituierte Carbonylgruppe, in der
$R_5$ ein Wasserstoffatom, eine Alkylgruppe mit 1 bis 6 Kohlenstoffatomen, die in 1-Stellung durch eine Morpholinocarbonyl-, Piperidinocarbonyl- oder Dimethylaminocarbonylgruppe und in 2-Stellung durch eine Morpholino- oder Pyrrolidinon-1-yl-Gruppe substituiert sein kann, eine Phenylmethyl- oder Pyridylmethylgruppe, eine Cycloalkyl- oder Cycloalkylmethylgruppe mit jeweils 5 oder 6 Kohlenstoffatomen im Cycloalkylteil, eine Menthyl- oder Norbornylgruppe darstellt,
oder eine $R_6$CO-O-CH$R_2$-O-CO-Gruppe darstellen, wobei
$R_2$ wie vorstehend erwähnt definiert ist und
$R_6$ eine Alkylgruppe mit 1 bis 4 Kohlenstoffatomen, eine Methoxy- oder Ethoxygruppe darstellt,
bedeuten,
deren Tautomere, deren Stereoisomere einschließlich deren Gemische und deren Additionssalze.

**3.** Carbonsäurederivate der allgemeinen Formel I gemäß Anspruch 1, in der
A eine Aminomethyl- oder eine Amidinogruppe, wobei in der Amidinogruppe an einem Stickstoffatom

ein Wasserstoffatom durch eine Methoxycarbonyl-, Ethoxycarbonyl- oder Benzyloxycarbonylgruppe ersetzt sein kann,
B eine gegebenenfalls durch ein Bromatom substituierte Phenylengruppe oder eine Pyridinylengruppe,
C eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sein können,
D eine Methylen-, Ethylen-, Carbonyl- oder Methylencarbonylgruppe, wobei die Carbonylgruppe der Methylencarbonylgruppe mit dem Rest E verknüpft ist, oder eine $-NR_3-CO-X$-Gruppe, in der das Stickstoffatom der $-NR_3-CO-X$-Gruppe mit dem Rest C verknüpft ist,
$R_3$ ein Wasserstoffatom oder eine Alkylgruppe mit 1 bis 3 Kohlenstoffatomen und
X eine geradkettige oder verzweigte Alkylengruppe mit 2 oder 3 Kohlenstoffatomen oder eine 1,4-Cyclohexylengruppe darstellen,
E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch eine >C=CH-Gruppe ersetzt sein kann, oder eine $-NR_4-X$-Gruppe, in der
X wie vorstehend erwähnt definiert ist und
$R_4$ ein Wasserstoffatom, eine Alkylgruppe mit 1 bis 5 Kohlenstoffatomen oder eine Phenylalkylgruppe mit 1 bis 3 Kohlenstoffatomen im Alkylteil darstellen,
F eine Bindung oder eine Methylengruppe und
G eine durch eine $R_5O$-Gruppe substituierte Carbonylgruppe, in der
$R_5$ ein Wasserstoffatom, eine Alkylgruppe mit 1 bis 5 Kohlenstoffatomen, die in 1-Stellung durch eine Morpholinocarbonyl-, Piperidinocarbonyl- oder Dimethylaminocarbonylgruppe substituiert sein kann, oder eine Cycloalkylgruppe mit 5 oder 6 Kohlenstoffatomen darstellt,
bedeuten,
deren Tautomere, deren Stereoisomere einschließlich deren Gemische und deren Additionssalze.

**4.** Carbonsäurederivate der allgemeinen Formel I gemäß Anspruch 1, in der
A und B zusammen eine 4-Aminomethyl-phenylgruppe, eine 4-Amidino-phenylgruppe, wobei in der Amidinogruppe an einem Stickstoffatom ein Wasserstoffatom durch eine Methoxycarbonyl- oder Benzyloxycarbonylgruppe ersetzt sein kann, oder eine 5-Amidino-pyrid-2-ylgruppe,
C eine 1,4-Cyclohexylengruppe, in der eine CH-Einheit in 1- oder 4-Stellung durch ein Stickstoffatom oder die CH-Einheiten in 1- und 4-Stellung jeweils durch ein Stickstoffatom ersetzt sind,
D eine Methylen-, Ethylen-, Carbonyl- oder Methylencarbonylgruppe, wobei die Carbonylgruppe der Methylencarbonylgruppe mit dem Rest E verknüpft ist, oder eine $-NR_3-CO-X$-Gruppe, in der das Stickstoffatom der $-NR_3-CO-X$-Gruppe mit dem Rest C verknüpft ist,
$R_3$ ein Wasserstoffatom oder eine Methylgruppe und
X eine geradkettige oder verzweigte Alkylengruppe mit 2 oder 3 Kohlenstoffatomen oder eine 1,4-Cyclohexylengruppe darstellen,
E eine 1,4-Cyclohexylen- oder 1,4-Cyclohex-3-enylengruppe, in denen die CH-Einheit in 1-Stellung, welche mit dem Rest D verknüpft ist, durch ein Stickstoffatom ersetzt ist und zusätzlich die CH-Einheit in einer 1,4-Cyclohexylengruppe in 4-Stellung, die mit dem Rest F oder G verknüpft ist, durch eine >C=CH-Gruppe ersetzt sein kann, oder eine $-NR_4-X$-Gruppe, in der
X wie vorstehend erwähnt definiert ist und
$R_4$ ein Wasserstoffatom, eine geradkettige Alkylgruppe mit 1 bis 5 Kohlenstoffatomen, eine Benzyl-, 2-Phenylethyl- oder 3-Phenyl-propylgruppe darstellen,
F eine Bindung oder eine Methylengruppe und
G eine durch eine $R_5O$-Gruppe substituierte Carbonylgruppe, in der
$R_5$ ein Wasserstoffatom, eine Alkylgruppe mit 1 bis 4 Kohlenstoffatomen, eine Methylgruppe, die durch eine Morpholinocarbonyl- oder Dimethylaminocarbonylgruppe substituiert ist, oder eine Cyclohexylgruppe darstellt,
bedeuten,
deren Stereoisomere einschließlich deren Gemische und deren Additionssalze.

**5.** Folgende Verbindungen der allgemeinen Formel I gemäß Anspruch 1:
(a) 1-(4-Amidinophenyl)-4-[N-(trans-4-carboxycyclohexyl)-aminocarbonyl]-piperazin,
(b) 1-(4-Amidinophenyl)-4-[N-(trans-4-carboxycyclohexyl)-N-methyl-aminocarbonyl]-piperazin,
(c) 1-(5-Amidinopyrid-2-yl)-4-[N-(trans-4-carboxycyclohexyl)-aminocarbonyl]-piperazin,
(d) 4-(4-Amidinophenyl)-1-[N-(trans-4-carboxycyclohexyl)-aminocarbonyl]-piperidin,

(e) 1-(4-Amidinophenyl)-4-[N-benzyl-N-(trans-4-carboxycyclohexyl)-aminocarbonyl]-piperazin,
(f) 1-(5-Amidinopyrid-2-yl)-4-[N-(trans-4-carboxycyclohexyl]-aminocarbonyl]-piperazin,
(g) 1-(5-Amidinopyrid-2-yl)-4-[N-(trans-4-carboxycyclohexyl]-N-methyl-aminocarbonyl]-piperazin,
(h) 4-(4-Aminomethylphenyl)-1-[N-(trans-4-carboxycyclohexyl)-aminocarbonyl]-piperidin,
(i) 4-(4-Amidinophenyl)-4-[4-(carboxymethyl)-piperidinocarbonyl]-piperazin,
(j) 1-(4-Amidinophenyl)-4-[4-(carboxymethyl)-3,4-dehydropiperidinocarbonyl]-piperazin,
(k) 1-(5-Aminomethylpyrid-2-yl)-4-[N-(trans-4-carboxycyclohexyl)-aminocarbonyl]-piperazin,
(l) 1-(5-Amidinopyrid-2-yl)-4-[4-(carboxymethyl)-piperidinocarbonyl]-piperidin,
(m) 1-(4-Aminomethylphenyl)-4-[N-(trans-4-carboxycyclohexyl)-aminocarbonyl]-piperazin,
(n) 1-[4-(N-Benzyloxycarbonyl-amidino)-phenyl]-4-[N-(trans-4-carboxycyclohexyl)-aminocarbonyl]-piperazin,
(o) 4-(4-Amidinophenyl)-1-[N-(trans-4-carboxycyclohexyl)-aminocarbonyl]-piperidin,
(p) 1-(4-Amidinophenyl)-4-[4-(carboxymethyl)-piperidinocarbonyl]-piperidin,
(q) 1-(4-Amidinophenyl)-4-[N-(2-carboxyethyl)-aminocarbonylmethyl]-piperidin,
(r) 1-(4-Amidinophenyl)-4-[N-(trans-4-carboxycyclohexyl)-aminocarbonyl]-piperidin,
(s) 1-(4-Aminomethylphenyl)-4-[N-(trans-4-carboxycyclohexyl)-aminocarbonyl]-piperidin,
(t) 1-(4-Amidinophenyl)-4-[N-(trans-4-carboxycyclohexyl)-N-(2-phenylethyl)-aminocarbonyl]-piperidin,
(u) 1-(4-Amidinophenyl)-4-[N-(trans-4-carboxycyclohexyl)-N-(3-phenylpropyl)-aminocarbonyl]-piperidin und
(v) 1-(4-Amidinophenyl)-4-[N-(trans-4-carboxycyclohexyl)-N-(n-pentyl)-aminocarbonyl]-piperidin,

deren Ester mit Cyclohexanol oder mit einem Alkanol mit 1 bis 4 Kohlenstoffatomen, wobei das Methanol durch eine Morpholinocarbonyl-oder Dimethylaminocarbonylgruppe substituiert sein kann, deren Stereoisomere einschließlich deren Gemische und deren Additionssalze.

**6.** Physiologisch verträgliche Additionssalze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Additionssalz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Carbonsäurederivate gemäß den Ansprüchen 1 bis 6, dadurch gekennzeichnet, daß

a) zur Herstellung von Verbindungen der allgemeinen Formel I, in der G eine Carboxygruppe darstellt, eine Verbindung der allgemeinen Formel

A - B - C - D - E - F - G'  ,(II)

in der
A, B, C, D, E und F wie in den Ansprüchen 1 bis 5 definiert sind und
G', das an ein Kohlenstoffatom gebunden ist, eine mittels Hydrolyse, Behandlung mit Säuren, Thermolyse oder Hydrogenolyse in eine Carboxygruppe überführbare Gruppe darstellt, in eine entsprechende Carboxyverbindung übergeführt wird oder

b) zur Herstellung von Verbindungen der allgemeinen Formel I, in der A eine Amidinogruppe darstellt, in der an einem der Stickstoffatome ein oder zwei Wasserstoffatome durch eine Alkyl- oder Phenylalkylgruppe ersetzt sein können, eine gegebenenfalls im Reaktionsgemisch gebildete Verbindung der allgemeinen Formel

$Z_1$ - C(=NH) - B - C - D - E - F - G  ,(III)

in der
B, C, D, E, F und G wie in den Ansprüchen 1 bis 5 definiert sind und
$Z_1$ eine Amino-, Alkoxy-, Aralkoxy-, Alkylthio- oder Aralkylthiogruppe darstellt, mit einem Amin der allgemeinen Formel

$R_a$ - NH - $R_b$ ,(IV)

in der
$R_a$ und $R_b$, die gleich oder verschieden sein können, Wasserstoffatome, Alkyl- oder Phenylalkylgruppen mit jeweils 1 bis 3 Kohlenstoffatome im Alkylteil bedeuten, umgesetzt wird oder
c) zur Herstellung von Verbindungen der allgemeinen Formel I, in der A eine Amidinogruppe darstellt eine Verbindung der allgemeinen Formel

NC - B - C - D - E - F - G ,(V)

in der
B, C, D, E, F und G wie in den Ansprüchen 1 bis 5 definiert sind, mit Hydroxylamin umgesetzt und anschließend eine so erhaltenes Amidoxim reduziert wird oder
d) zur Herstellung von Verbindungen der allgemeinen Formel I, in der A eine Amidinogruppe darstellt, in der an einem der Stickstoffatome ein oder zwei Wasserstoffatome durch eine Alkyl- oder Phenylalkylgruppe ersetzt sein können, eine Verbindung der allgemeinen Formel

NC - B - C - D - E - F - G ,(V)

in der
B, C, D, E, F und G wie in den Ansprüchen 1 bis 5 definiert sind, mit einem entsprechenden Alkylchloroaluminiumamid umgesetzt und eine so erhaltene Verbindung anschließend hydrolysiert wird oder
e) zur Herstellung von Verbindungen der allgemeinen Formel I, in der A eine geradkettige oder verzweigte Aminoalkylgruppe mit 1 bis 3 Kohlenstoffatomen darstellt, eine Verbindung der allgemeinen Formel

$A_1$ - B - C - D - E - F - G ,(VI)

in der
B, C, D, E, F und G wie in den Ansprüchen 1 bis 5 definiert sind und
$A_1$ eine Cyano-, Cyanomethyl-, 1-Cyanoethyl- oder 2-Cyanoethylgruppe darstellt, reduziert wird oder
f) zur Herstellung von Verbindungen der allgemeinen Formel I, in der A eine geradkettige oder verzweigte Aminoalkylgruppe mit 1 bis 3 Kohlenstoffatomen, eine Amino-, Amidino- oder Guanidinogruppe, wobei in jeder der vorstehend erwähnten Gruppen jeweils an einem der Stickstoffatome ein Wasserstoffatom durch eine Alkoxycarbonylgruppe mit insgesamt 2 bis 5 Kohlenstoffatomen, durch eine Benzyloxycarbonyl-, Allyloxycarbonyl-oder $R_1$-CO-O-($R_2$CH)-O-CO-Gruppe ersetzt ist, darstellt, wobei $R_1$ und $R_2$ wie in den Ansprüchen 1 bis 5 definiert sind, eine Verbindung der allgemeinen Formel

$A_2$ - B - C - D - E - F - G ,(VII)

in der
B, C, D, E, F und G wie in den Ansprüchen 1 bis 5 definiert sind und
$A_2$ eine geradkettige oder verzweigte Aminoalkylgruppe mit 1 bis 3 Kohlenstoffatomen, eine Amino-, Amidino- oder Guanidinogruppe darstellt, mit einer Verbindung der allgemeinen Formel

$Z_2$ - $R_c$ ,(VIII)

in der
$R_c$ eine Alkoxycarbonylgruppe mit insgesamt 2 bis 5 Kohlenstoffatomen, eine Benzyloxycarbonyl-, Allyloxycarbonyl- oder $R_1$-CO-O-($R_2$CH)-O-CO-Gruppe, wobei $R_1$ und $R_2$ wie in den Ansprüchen 1 bis 5 definiert sind, und

$Z_2$ eine nukleophile Austrittsgruppe darstellen, umgesetzt wird oder
g) zur Herstellung von Verbindungen der allgemeinen Formel I, in der G eine durch eine $R_5$'O-Gruppe substituierte Carbonylgruppe darstellt, wobei $R_5$' mit Ausnahme des Wasserstoffatoms die für $R_5$ in den Ansprüchen 1 bis 5 erwähnten Bedeutungen besitzt, eine Verbindung der allgemeinen Formel

A - B - C - D - E - F - $G_1$ ,(IX)

in der
A, B, C, D, E und F wie in den Ansprüchen 1 bis 5 definiert sind und
$G_1$ eine Carboxy- oder Alkoxycarbonylgruppe darstellt, mit einem Alkohol der allgemeinen Formel

HO - $R_5$' ,(X)

in der
$R_5$' mit Ausnahme des Wasserstoffatoms die für $R_5$ in den Ansprüchen 1 bis 5 erwähnten Bedeutungen besitzt, umgesetzt wird oder
h) zur Herstellung von Verbindungen der allgemeinen Formel I, in der G eine durch eine $R_5$'O-Gruppe substituierte Carbonylgruppe oder eine $R_6$CO-O-CHR$_2$-O-CO-Gruppe darstellt,
wobei $R_2$ und $R_6$ wie in den Ansprüchen 1 bis 5 erwähnt definiert sind und $R_5$' mit Ausnahme des Wasserstoffatoms die für $R_5$ in den Ansprüchen 1 bis 5 erwähnten Bedeutungen besitzt, eine Verbindung der allgemeinen Formel

A - B - C - D - E - F - COOH ,(XI)

in der
A, B, C, D, E und F wie in den Ansprüchen 1 bis 5 definiert sind, mit einer Verbindung der allgemeinen Formel

$Z_3$ - $R_d$ ,(XII)

in der
$R_d$ mit Ausnahme des Wasserstoffatoms die für $R_5$ in den Ansprüchen 1 bis 5 erwähnten Bedeutungen besitzt oder eine $R_6$CO-O-CHR$_2$-Gruppe darstellt, wobei $R_2$ und $R_6$ wie in den Ansprüchen 1 bis 5 erwähnt definiert sind und
$Z_3$ eine nukleophile Austrittsgruppe darstellt, umgesetzt wird und
erforderlichenfalls ein während den Umsetzungen zum Schutze von reaktiven Gruppen verwendeter Schutzrest abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.